# EUROPEAN PATENT APPLICATION

(11) **EP 2 620 126 A1**
(43) Date of publication of application: **31.07.2013**
(21) Application number: 13163640.9
(22) Date of filing: 18.02.2011
(51) Int. Cl.: A61F 2/66

(54) **Joints for prosthetic, orthotic and/or robotic devices**

(30) Priority: 19.02.2010 US 306404 P
(62) Divisional of application: 11707266.0
(71) Applicant: Tensegrity Prosthetics Inc., Louisville, CO 80027 (US); Keane, Jerome, Louisville, Colorado 80027 (US)
(72) Inventor: Keane, Jerome, Louisville, CO 80027 (US); Rifkin, Jerome R., Louisville, CO 80027 (US)
(74) Representative: Moore, Barry

(57) **Abstract**

An artificial foot device, comprising: a talus body; a core pivotably coupled with the talus body at a first joint; and a biasing assembly (290) acting between the talus and the core at the first joint; wherein the talus body pivots about the first joint and causes the biasing assembly to provide a torque response for the artificial foot device; the first joint comprises a lateral axle (124a) extending from the talus body; pivot bearings are coupled to the lateral axle; and the talus body pivots about the pivot bearings and the pivot bearings slide along the core.

## Description

### REFERENCE TO RELATED APPLICATIONS

This application claims priority to U.S. provisional Patent Application No. 61/306,404, filed February 19, 2010, entitled "Prosthetic Foot", which is incorporated herein in its entirety by reference.

This application is related to pending U.S. Patent Application No. 12/464,747, filed May 12, 2009, which claims the benefit of priority of provisional U.S. Patent Application No. 61/127,482, filed May 13, 2008, the entirety of which are incorporated herein by reference.

This application is also related to pending U.S. Patent Application No. 11/080,972, filed March 16, 2005, which claims the benefit of priority of provisional U.S. Patent Application No. 60/553,619, filed March 16, 2004, the entirety of which are incorporated herein by reference.

This application also claims the benefit of priority and is a continuation-in-part ("CIP") of U.S. nonprovisional application 12/630,934, filed December 4, 2009, which claims the benefit of priority of provisional U.S. Patent Application No. 61/225,439, filed July 14, 2009, the entirety of which are incorporated herein by reference.

### TECHNICAL FIELD

This application relates generally to artificial foot devices, such as a prosthetic, orthotic or robotic foot that simulates the coordinated motions of the natural human foot, particularly in walking gait. More particularly, embodiments of this application relate to a prosthetic, orthotic or robotic foot including three segments connected by two joints: one joint analogous to the human first metatarsophalangeal joint, and the other joint analogous to the human subtalar joint.

### BACKGROUND

People who lose a leg today may be in a bad situation. Some days, a simple staircase may seem like an insurmountable challenge. Walking up a grassy slope is too difficult to attempt, because multiple falls may be inevitable. War, accidents and disease keep this disadvantaged population growing. Prosthetics, or synthetic replacements for missing anatomical structures, hold the promise of restoring some of this lost function and improving quality of life.

Just trying to regain functional mobility, amputees spend an average of $8,000 on below-knee (BK) prosthetic legs that last three to five years. Rather than spend this money on costly, non-repairable devices, one hundred and twenty thousand American amputees have chosen crutches or wheelchairs, and they won't walk again.

Just as the speed of a vehicle is maintained through regular energetic pushes received from pistons firing in the engine, normal human gait relies on well-timed pushes from the anatomy of the foot, during the toe-off portion of the walking cycle. Providing a suitable timing of toe-off, while providing a stable, level base - a preferable innovation addressed in this application - is lacking from existing feet prostheses and may be relevant for natural and comfortable ambulation.

The human gait is in reality a very complex process that at a basic level may be described as a series of repeating operations carried out by a single leg: 1) initial heel strike, 2) double support as both feet contact the ground, 3) stance phase as one leg supports the entire body weight, 4) pre-swing or heel-rise as the heel rises from the ground, 5) toe-off as the moment that the toes lose contact with the ground, and finally 6) swing phase, where the leg, acting as a pendulum, comes forward in preparation to repeat the process. In a two legged description of pre-swing, the heel of the contralateral leg strikes the ground at the exact moment that the ipsilateral heel rises. This is called double stance phase, and may be relevant to understanding the innovations presented in this application. Coordinated movement between the legs and the overall balance and trajectory of the body dynamic may be also relevant to successful ambulation.

Currently, there are two dominant paradigms of prosthetic foot design: post-like, conventional feet (CF) and leaf-spring-like, energy storing feet (ESF). Both of these designs change shape under loading, in an attempt to mimic the human foot. The classic CF foot, also known as the Solid Ankle Cushioned Heel (SACH), foot may provide a stable base for support, and is functionally unchanged since its conception in the 1960's. Introduced in the 1980's, carbon-fiber, leaf-spring ESF designs allow amputees to run by mimicking the ankle plantar flexors, returning energy to their stride. Para-lympic records rivaling their Olympic counterparts show that the ESF paradigm works very well for running, but studies have failed to show that these benefits extend to walking. 40% of transtibial amputees do not use prostheses and 78% of transfemoral amputees forego this intervention. Thus, over 120,000 amputees do not use prosthetic legs, preferring wheelchairs or crutches, never walking again. Studies of amputee psychosocial adjustment have linked positive emotional coping and higher levels of physical independence.

Depending on the type of foot used, CF or ESF, and the specific manufacturer, there have been subtle but significant differences in parameters such as stride length, symmetry of stride, and timing of the various phases of gait. For either foot type, stride length is shorter for strides where the prosthesis is the supporting limb, gait symmetry is markedly decreased, and the timing of the phases of gait may be disrupted. Most notably, there is a shortened stance phase on the prosthesis, a late toe off, and a longer swing phase on the affected side as well. Studies also describe an early incidence of low back and patellar-femoral osteoarthritis in unilateral amputees. The literature clearly shows that current prostheses fail to walk like an intact limb. In fact, clinical prostheticists have expressed the opinion that some "middle ground" between the unsophisticated CF feet and the highly athletic ESF feet is needed. Embodiments of the invention outlined here may be just that middle ground.

To lay the foundation for the rest of this submission, a few questions may be asked. Precisely how may an intact limb walk? And what is the role of the foot in this process? To address the first question, this application may present two different types of engineering control systems, and may provide illustrative examples. To address the second question, more studies may be presented, furthering the discussion, showing results of highly detailed, instrumented gait studies of the foot. Comparisons between the functional movements of the human foot, and the functional movements of current prostheses may follow. The improvements embodied in embodiments of the proposed device may address many of the shortcomings seen in the current technology.

With all of the myriad muscles and bones in human hips, legs, and feet, there is no "right" answer for how to propel one's self across a room or up a slope; however, there may be more optimal solutions, for example, ones that may be less abusive to the anatomy and/or ones with more optimal energetic efficiency. Early incidence of osteoarthritis, a degenerative joint change, is one indicator of a suboptimal movement strategy.

There may be many ways to walk, and data shows that people don't walk in exactly the same way with each stride. The hips may work harder on some strides than others; sometimes the lower leg may contribute varying amounts torque to the stride. Walking from one's hips may be described as a "top-down" control mechanism, where forces from the proximal leg may dictate the position and accelerations of the distal structures. This mechanism is very clearly illustrated in above knee (AK) amputees. Until recent, expensive innovation of computer controlled knees, AK amputees who wanted to walk faster than the return rate of their knee spring had to use a "hip snap," flinging their prosthesis out quickly with their hip flexors, and then quickly contracting their hip extensors to snap the prosthetic knee straight in time for heel-strike. Thus, the anatomic ranges of motion guided the position of the prosthetic anatomy, but the timing the movement was controlled by the hip, in a "top-down" fashion.

A "top-down" control mechanism may also be seen in studies of trans-tibial amputees. The iEMG data of one study showed a greater use of the biceps femoris (BF) as compared to the antagonistic vastus medialus (VM) in the amputated limb, as opposed to the normal limb. The mean ratios of BF/VM activity during the first half of stance phase was 3.8 in the amputated limb and 2.0 in the sound leg, with a P value of less than 0.042. Furthering elaboration on the "top-down" nature of this control system, an exceptionally statistically rigorous study from 2002 revealed some interesting trends in the flexor/extensor ratios for the knees of unilateral, trans-tibial amputees, as compared to normal volunteers. Though the amputees were much weaker than the normal control group, this study showed that there was no significant difference between the knee flexor/extensor ratios for peak bending moment, total work, or maximum power comparing either leg of the amputees and either leg of the non-amputees. Of course, the BF and VM may be also knee flexors and extensors, but not during the relevant time-span cited by the first study, early stance phase. Considering these studies together, one may conclude that trans-tibial amputees use the hip of the amputated leg more than the hip of their sound leg, and that they use their knee flexors and extensors normally. Clearly, the control mechanism being employed in a trans-tibially amputated limb is "top-down."

The overuse of a particular muscle must result in overuse of the surrounding and supporting muscles. For example over loading a hip muscle causes the hip stabilizers to be over-recruited. If multifidus and transversus abdominus, the deepest pelvic stabilizers, may be overwhelmed, the larger quadratus lomborum (OL) and erector spinae (ES) muscles that may be normally used for motion may be recruited to help it. When the QL and ES are used as stabilizers, the agonists may also be recruited as stabilizers, just as transverses abdominus is recruited along with multifidus. When the QL and ES become a routine part of the stabilizing muscle pattern, they become tonic and rigid. Thus, putting a great deal of compression on the spine. This is a well-known pattern of muscle use and, if allowed to progress unchecked, may eventually result in degenerative joint changes in the lower spine.

Walking from the foot, as opposed to the hip, may be modeled as a "bottom-up" control scheme, where the distal anatomy directs the position of the proximal anatomy. The coordination of the metatarsophalangeal joint (MTP) of the great toe and the subtalar joint may create a dynamic in gait where the proximal foot and tibia subtly change angular position. This angular change may be the start of building momentum for toe off. In context of the gait cycle, starting from single stance phase, as the tibial shaft moves past perpendicular and over the foot, the subtalar joint may be eccentrically loaded. This may be seen as a "flatter" transverse arch. This subtle motion may progress with the tibial shaft advancement, with a maximum angular change of 10 degrees. In double stance phase, much of this weight may be off-loaded to the other leg, but the transverse arch may not yet spring back into shape. In fact, this new conformation may be maintained until just after heel rise. When the heel leaves the ground, passing the remaining force loading to the ball of the great toe, the MTP of the great toe may be forced into extension. This motion may pull on the plantar aponeurosis, which in turn may pull on the calmayeus and the Achilles tendon. This action may loft the transverse arch back to its stance phase conformation, subtly altering the position of the ankle and the tibia, and thus may change position of the knee and hip.

The relevant anatomy for this coordination of the first MTP and subtalar joints is well documented. The plantar aponeurosis spans both joints, as may the tendon of the flexor hallucis longus. Different research references attribute this coordination to each of these sources. The action of arching the subtalar joint by forcibly extending the first MTP has been described as the Windlass mechanism, and this passive, non-muscular change may be a function of timing and anatomic length. This timing may be influenced by the peronii, the tibialis anterior, and the intrinsic foot muscles. Of course, a passive prosthesis may not duplicate the action of these muscles, but it may mimic the action of the plantar aponeurosis. Due to the quasi-psuedoviscoelastic nature of the plantar aponeurosis and the surrounding musculature, this quick lofting of the plantar arch may be an energy storage mechanism. The energy may then be released, a moment later, on toe off. As seen in the temporal gait asymmetry of amputees, most notably in late stance and swing phases, studies have shown conclusively that this action is not accomplished in either CF or ESF designs.

These two distinct "ways of walking" represent extremes, and, as human nature dictates, we all walk with a varying degree of each mechanism. Amputees must rely exclusively on the strategy of top-down control, resulting in an overcompensation of the remaining anatomy which in turn may cause early degenerative changes. What is needed is a prosthesis that accurately imitates the relevant biomechanics of the natural foot, allowing for the contributions of the more efficient "bottom-up" gait style.

There is a definite coordination between the joints of the foot. The angular relationship shown between the forefoot and hallux may be the angular position of the first MTP. The angular motion between the forefoot and hindfoot may reflect the motion of the subtalar joint. A few studies have explored the detailed biomechanics of the foot using this powerful analytical technique, but they did not combine the detailed foot analysis with the protocol for the rest of the body. Thus, no quantified joint powers were generated. Experts may also be aware of the subtle, but highly significant errors in instrumented gait analysis of ESF prosthesis gait. Failure to accurately model the center of curvature of the leaf spring foot, for the purpose of reverse engineering the joint torques, may be the documented source of this error. The standard seven segment lower body model, used to reverse engineer joint torques, may use a rigid single segment foot. This simplified model may leave out both the first MTP and the subtalar joints, masking the relevant contribution of the Windlass mechanism, a subtle "bottom-up" contributor of gait mechanics. Theoretically, a nine segment lower body model, as seen in computer simulations, may show sensitivity to changes in spring stiffness of the MTP joint at push off, but still may exclude the subtalar joint or any coordination of the two.

The movement of the subtalar joint and first MTP during stance phase and toe-off, as described above, may correlate to a relatively new area of prosthetics research. Roll-over shape may be defined as the geometry a foot/ankle complex takes during the single limb stance phase of walking. As the center of weight may pass over the long axis of the prosthetic foot, it may bend according to its stiffness. The shape described by this bending may be the rollover-shape, and it may be defined in general terms as a rigid rocker model of the foot/ankle complex. A three dimensional rollover shape may be called a rollover surface, and a two dimensional shape may be called a rollover profile.

Studies of various prosthetic feet with the rollover profile methodology have shown that the "effective foot length" during walking is surprisingly short in many cases. For example, a size 28 cm SACH foot may display a functional length of less than 20 cm. The length of the rollover profile is significant for many gait parameters, and recent studies show that it may be relevant to how much oxygen is consumed during gait.

Considering the rollover profile length, along with the recent research into oxygen consumption dynamics, points toward a discrepancy that may be more significant than previously thought. In fact, the energy used in walking may be proportional to the fourth power of the step length. Since the stride length may be equal to the functional foot length plus the distance covered by swing phase, feet with shorter rollover profiles may deliver shorter stride lengths. The average step length is about 0.75 meters, and the difference in rollover profile between a SACH foot and a flex-foot is about 6 centimeters. Considering the relationship described above, one would anticipate a large energy savings by using the longer flex-foot, because the step length is almost 10% greater for the ESF versus over the CF. Surprisingly, this energy savings is not seen in any ESF models with longer rollover profiles. In fact, research shows a small energy savings, on the order of 3%, and some of the research subjects in that study found that some ESF feet were more tiring to use than some CF feet. This correlates well with the experience of clinical prosthetists, who describe that their patients often work against their ESF feet, because their return of power is not biomechanically accurate. Indeed, studies of prostheses show that a very small component of this energy return is in the antero-posterior direction, unlike the natural human limb.

### SUMMARY

There is a need for improved artificial foot devices. In particular, there is a need for artificial foot devices that more accurately simulate the motion and or function of the human foot during walking.

In one embodiment, an artificial foot device may be provided. The artificial foot device may include: a toe pivotably coupled with a core at a joint and, a vertical restraint link extending across the joint that couples the toe with the core. The vertical restraint link limits motion of the toe in a vertical direction relative to the core.

In another embodiment, an artificial foot device may include a talus body, a core pivotably coupled with the talus body at a first joint, a toe pivotably coupled with the core at a second joint, and a tension member coupling the toe to the talus body.

In another embodiment of an artificial foot device, the artificial foot device may include a talus body, a core pivotably coupled with the talus body at a first joint, and a biasing assembly acting between the talus and the core at the first joint. The talus body pivots about the first joint and causes the biasing assembly to provide a torque response for the artificial foot device.

In another embodiment, an artificial foot device may be provided that includes a talus body, a core operatively coupled with the talus body by a first joint that provides for constrained relative movement between the talus body and the core; and a toe operatively coupled with the core by a second joint that provides for constrained relative movement between the core and the toe.

In some embodiments, the first joint may permit limited relative rotation of the talus body and the core about a first lateral axis, with the talus body, the core and the toe defining a longitudinal direction of the artificial foot. Alternatively or additionally, the first joint may permit limited relative rotation of the talus body and the core about a first longitudinal axis. Alternatively or additionally, the first joint may permit limited relative rotation of the talus body and the core about a substantially vertical axis.

In some embodiments, the second joint may permit limited relative rotation of the core and the toe about a first lateral axis. Alternatively or additionally, the second joint may permit limited relative rotation of the core and the toe about a first longitudinal axis. Alternatively or additionally, the second joint may permit limited relative rotation of the core and the toe about a substantially vertical axis. Alternatively or additionally, the second joint may permit limited relative lateral movement between the core and the toe.

In some embodiments, the first joint may include means for constraining relative movement between the talus body and the core other than about a lateral axis. In such embodiments, the means for constraining relative movement between the talus body and the core other than about a lateral axis may be configured to constrain relative movement between the talus body and the core about a longitudinal axis and/or about a substantially vertical axis.

In some embodiments, the second joint may include means for constraining relative movement between the core and the toe other than about a lateral axis. In such embodiments, the means for constraining relative movement between the core and the toe other than about a lateral axis may be configured to constrain relative movement between the core and the toe about a longitudinal axis and/or about a substantially vertical axis.

In some embodiments, the constrained relative movement between the talus body and the core may substantially correspond to a coordinated movement of a first natural joint and a second natural joint during ambulation of a natural human foot. In such embodiments, the constrained relative movement between the core and the toe may substantially correspond to a coordinated movement of a third natural joint, different from the first and second natural joints, during ambulation of a natural human foot.

In some embodiments, the artificial foot may include a coordination member operatively coupled with the talus body and the toe. In such embodiments, the coordination member may be configured to store and release energy during a walking movement of the artificial foot.

In some embodiments, the artificial foot may include a coordination member operatively coupled with the talus body and the core. In such embodiments, the coordination member may be configured to store and release energy during a walking movement of the artificial foot.

In some embodiments, the artificial foot may include at least one member operatively coupled with the core and the toe. In such embodiments, the at least one member may be configured to store and release energy during a walking movement of the artificial foot.

In some embodiments, a method of providing motion in an artificial foot device may be provided. The method may include coordinated movements of three or more structural members operatively coupled by two or more joints. The coordinated movements may be constrained by the two or more joints and/or interactions between the three or more structural members.

In some embodiments, a method of providing motion in an artificial foot device may include constrained relative movement between a first structural member and a second structural member. Such constrained movement may substantially correspond to a coordinated movement of a first natural joint and a second natural joint during ambulation of a natural human foot.

In such embodiments, the method may include constrained relative movement between the second structural member and a third structural member. Such constrained movement may substantially correspond to a coordinated movement of a third natural joint, different from the first and second natural joints, during ambulation of a natural human foot.

### BRIEF DESCRIPTION OF THE DRAWINGS

These and other features, aspects and advantages will be more fully understood when considered with respect to the following detailed description, appended claims and accompanying drawings, wherein:

FIGS. 1-6 illustrate an artificial foot including a talus body, a core and a toe according to an embodiment.

FIG. 7 illustrates an exploded view of the artificial foot illustrated in FIGS. 1-6.

FIG. 8 is a complete assembly of the artificial foot illustrated in FIGS. 1-7.

FIGS. 9-13 illustrate views of the talus body of the artificial foot illustrated in FIGS. 1-8.

FIGS. 14-19 illustrate views of the core of the artificial foot illustrated in FIGS. 1-8.

FIGS. 20-26 illustrate views of the toe of the artificial foot illustrated in FIGS. 1-8.

FIGS. 27-30 illustrate views of the encasement of the core spring carrier assembly of the artificial foot illustrated in FIGS. 1-8.

FIGS. 31-35 illustrate views of the encasement of the toe spring carrier assembly of the artificial foot illustrated in FIGS. 1-8.

FIGS. 36-38 illustrate views of one of the links for coupling the core and toe of the artificial foot illustrated in FIGS. 1-8.

FIGS. 39-40 illustrate views of an alternative link for coupling the core and toe of the artificial foot illustrated in FIGS. 1-8.

FIG. 41 illustrates an artificial foot including a talus body, a core and a toe according to another embodiment.

FIGS. 42-46 illustrate a toe and a core of the artificial foot illustrated in FIG. 41.

FIG. 47a illustrates an exploded view of the artificial foot illustrated in FIG. 41.

FIG. 47b illustrates a view of a pivot bearing and pivot bumper for use with the artificial foot illustrated in FIG. 41.

FIGS. 48-49 illustrate the core of the artificial foot illustrated in FIGS. 41 and 47.

FIGS. 50-52 illustrate the core and links of the artificial foot illustrated in FIGS. 41 and 47.

FIGS. 53a-53b illustrate a partial section of the core and links in an alternative configuration that may be used in connection with the artificial foot illustrated in FIGS. 1-8 and 41 and 47.

FIGS. 54a-54c illustrate the toe that may be used in connection with the core and links illustrated in FIGS. 53a-53b.

FIG. 54d illustrates the assembly of the toe and core and the links provided in FIGS. 53a to 54c.

FIGS. 55-61 illustrate the toe and the toe insert of the artificial foot illustrated in FIGS. 41 and 47.

FIGS. 62-68 illustrate the toe insert for incorporation with the toe of the artificial foot illustrated in FIGS. 47 and 47.

FIGS. 69-75 illustrate the toe, the toe insert and the toe bushing of the artificial foot illustrated in FIGS. 41 and 47.

FIGS. 76-81 illustrate the toe bushing for incorporation with the toe and toe insert of the artificial foot illustrated in FIGS. 41 and 47.

FIG. 82 illustrates a torque response curve.

### DETAILED DESCRIPTION

Various details are described below, with reference to illustrative embodiments. It will be apparent that the invention may be embodied in a wide variety of forms, some of which may be quite different from those of the disclosed embodiments. Consequently, the specific structural and/or functional details disclosed herein are merely representative and do not limit the scope of the invention.

For example, based on the teachings herein it should be understood that the various structural and/or functional details disclosed herein may be incorporated in an embodiment independently of any other structural and/or functional details. Thus, an apparatus may be implemented and/or a method practiced using any number of the structural and/or functional details set forth in the disclosed embodiments. Also, an apparatus may be implemented and/or a method practiced using other structural and/or functional details in addition to or other than the structural and/or functional details set forth in the disclosed embodiments.

Embodiments of the device proposed in this application may be based on the tensegrity design idea. Tensile-integrity, shortened to "tensegrity," may refer to a special type of structure comprising continuous tensile members, such as cables, acting upon discontinuous compressive members (e.g., spars). Tensegrity structures may rely upon the tensile strength and flexibility properties of wire rope to bear physical loads placed upon them. Major innovations in steel wire rope technology, driven by increasing performance demands in the automotive and aerospace sectors, now permit the construction of light weight joints that may be stronger in many cases than traditional engineered "beam and bearing" structures. Also, a solid link, such as a rod, that is not free to rotate to an orientation of pure tension may be employed.

As used herein, "tensegrity" may refer to the characteristic of having two or more discontinuous members dispersed in a network of one or more continuous tension members.

As used herein, "tensegrity joint" may refer to a joint having a tensegrity structure. In a tensegrity joint, the two or more discontinuous members may be incompletely constrained by the network of the one or more continuous tension members in which they may be dispersed, whereby the members may be able to move relative to each other. The movement may be at one or more centers of motion, and optionally around a primary axis at each center. Optionally, the primary axis may be virtual and not coaxial with an actual tension member. As described herein, in addition to the constrained or limited range of relative motion of the discontinuous members of the joint provided by the tension member(s), contact between the discontinuous members may also constrain or limit the relative motion.

As referred herein, dorsiflexion may be defined as motion in the direction of the top of the foot (e.g., dorsal surface), and plantarflexion may be defined as motion in the direction of the bottom of the foot (e.g., plantar surface).

In a tensegrity joint, the discontinuous members may be rigid, and the number, length, diameter, geometric organization, and flexibility characteristics of the tension members may determine the range of motion of the discontinuous members. Tension members may constrain and/or stabilize the discontinuous members.

Further, a tensegrity approach may be employed to couple movements of multiple joints. For example, the movement or motion of a first joint may be coupled to movement/motion of a second joint via one or more tension members, such as a coupling cable. The coupling cable(s) may connect two discontinuous members that may not be connected by a single joint, but indirectly connected by two or more joints.

Each joint in the animal body may have its own specific geometry. Joint(s) in embodiments of the invention may be designed to have similar characteristics of natural joints. Alternatively, super joints may be designed for prosthetics, orthotics, and robotics that do not interfere with the functioning of the remaining joints of the body or robot.

The materials of the discontinuous and tension members may be selected to maintain structural integrity considering the use of the device and the user of the device. Devices that must withstand greater forces may be made from stronger materials.

As used herein, "rope" may refer to an element capable of functioning as a tension member in a tensegrity joint, such as cables having a diameter less than about 1/4 inch, for example. An effective rope element may comprise two or more thinner ropes, including a thin rope making multiple passes and having a larger effective diameter than the thin rope alone. Generally, these ropes may not be elastic.

As used herein, "rod" may refer to an element capable of functioning as a tension member in a tensegrity joint as well. It should be understood that the rod described in the particular embodiments herein is only an example.

Orthotics may augment body parts. Prosthetics may replace body parts. Robotics may function similarly to body parts, but may not require direct connection to a body in order to be functional.

Limit ropes and stabilization ropes may be tension members in tensegrity joints that limit the ranges of motion of the joint. Optionally, the tensegrity joint may have a primary axis of motion. A primary axis of motion may be the least constrained axis of all the other axes of motion of the joint. Optionally, one tension member may be coaxial with the primary axis of motion of the joint. Alternatively, the primary axis of motion may not be coaxial with a tension member, and the primary axis may then be said to be virtual.

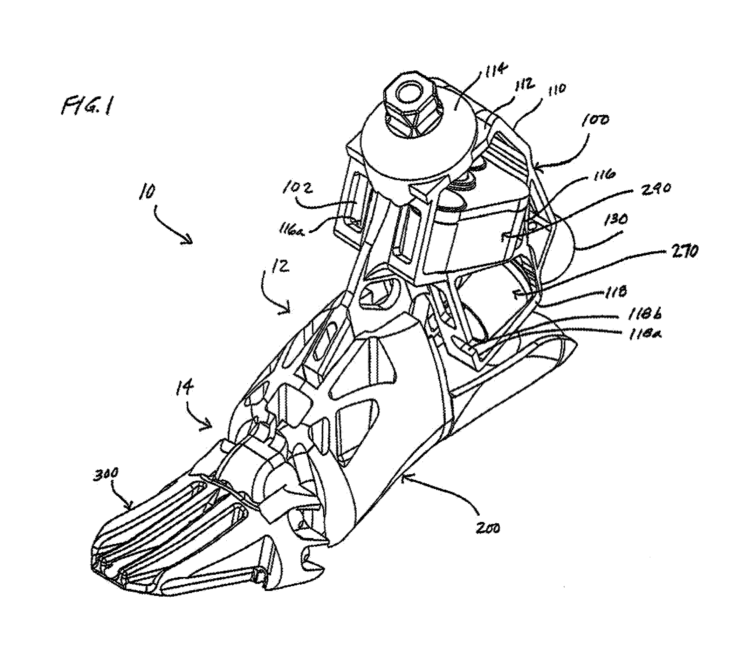

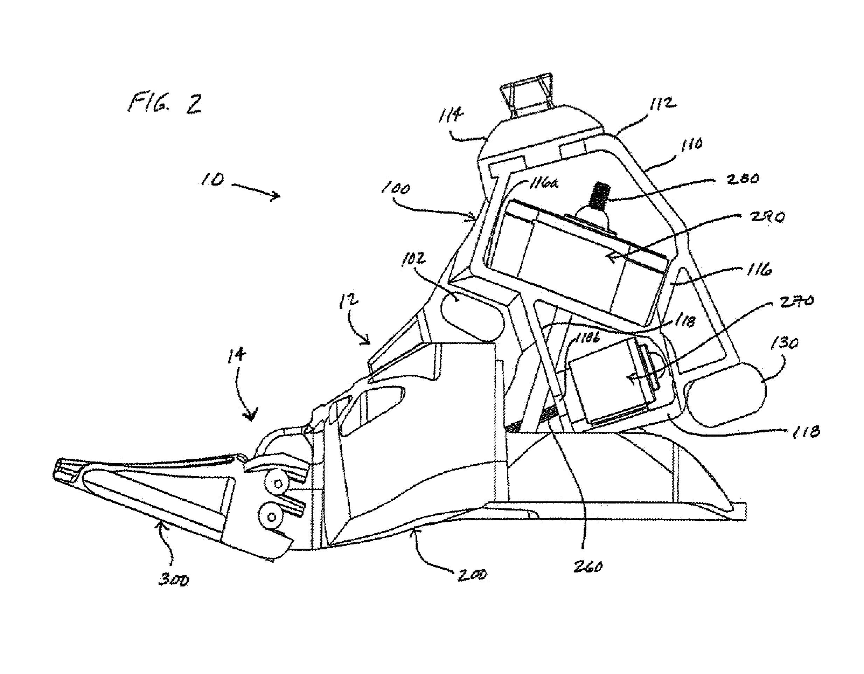

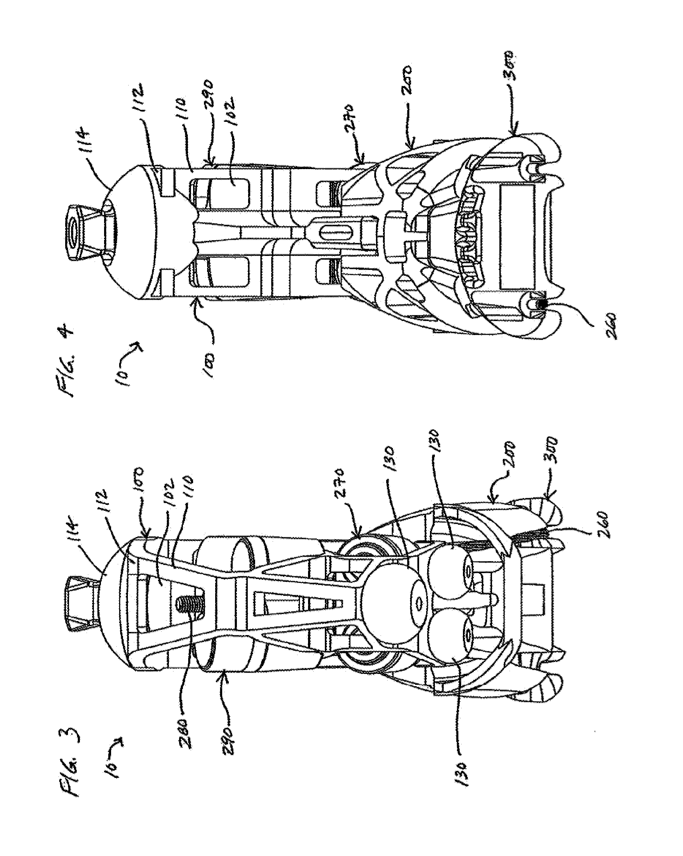

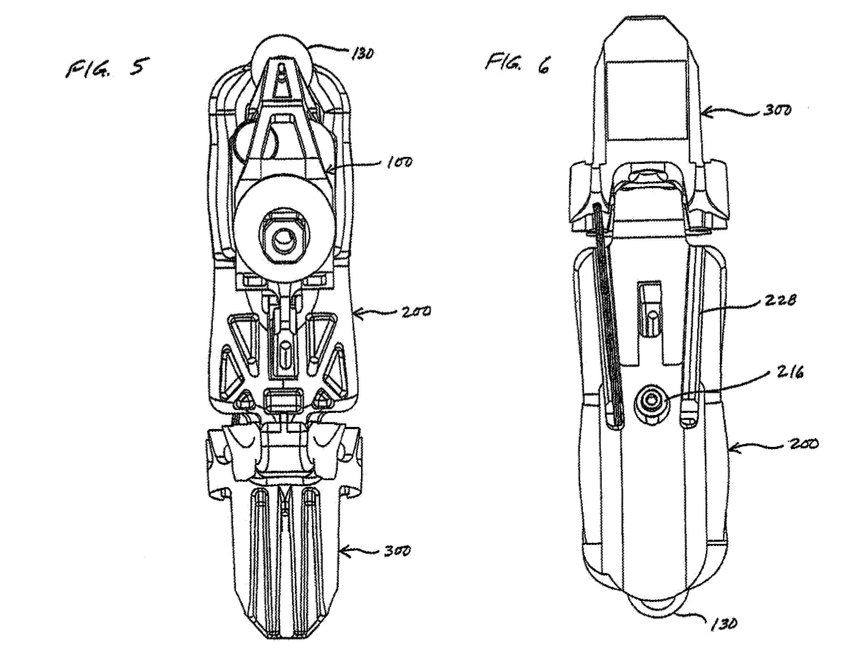

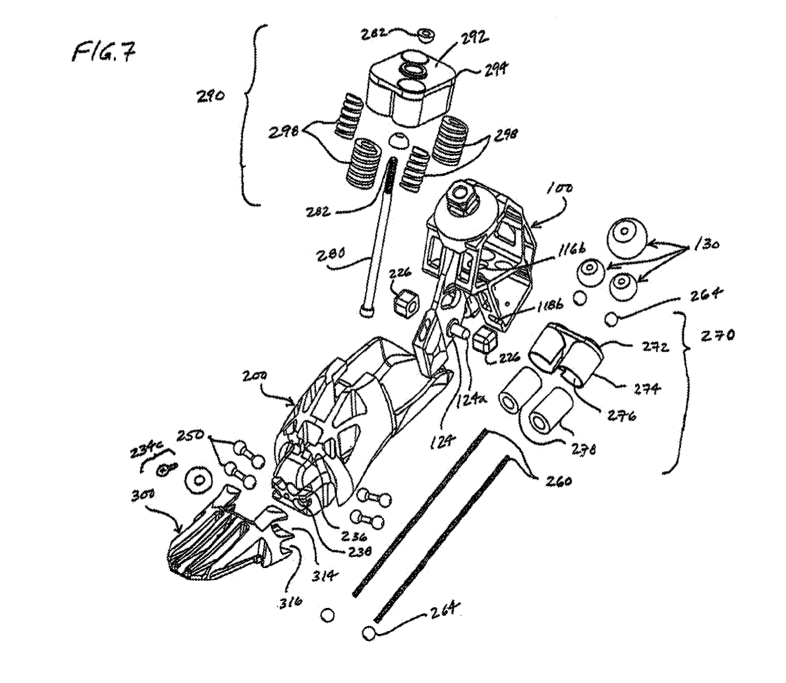

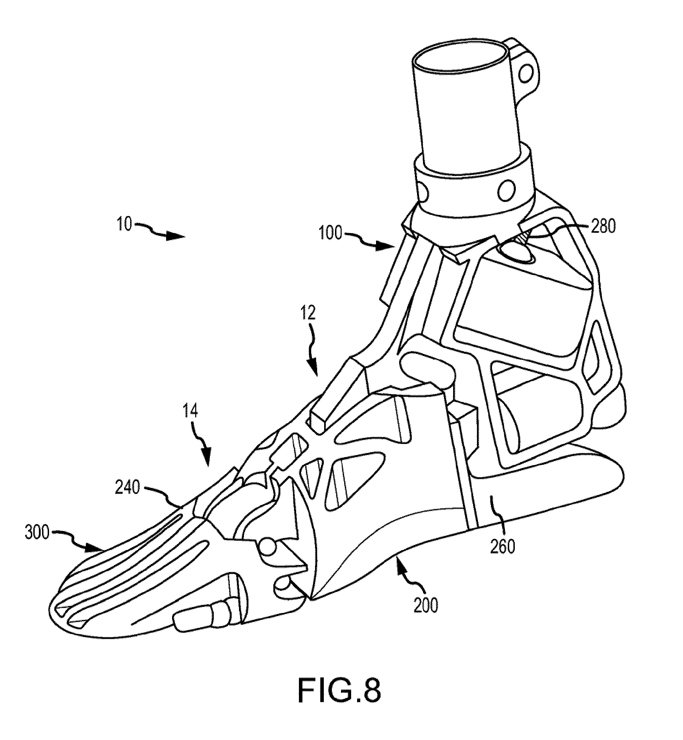

FIGS. 1-8 illustrate an artificial foot 10 including a talus body 100, a core 200 and a toe 300 according to an embodiment of the invention. FIG. 1 depicts a front perspective view of the artificial foot 10. FIG. 2 depicts a left side view of the artificial foot 10, the right side view of the artificial foot 10 being identical in this embodiment. FIG. 3 depicts a rear view of the artificial foot 10. FIG. 4 depicts a front view of the artificial foot 10. FIG. 5 depicts a top view of the artificial foot 10. FIG. 6 depicts a bottom view of the artificial foot 10. FIG. 7 is an exploded view of the artificial foot 10. FIG. 8 is a complete assembly of the artificial foot 10.

The talus body 100 may be operatively coupled with the core 200 via a first joint 12. The core 200 may be operatively coupled with the toe 300 via a second joint 14. As described herein, it should be understood that the first and second joints 12, 14 include one or more tension/limit members, such as ropes, some of which are not shown in these FIGS. for simplicity. As discussed above, the tension/limit member(s) may constrain movement of the talus body 100, the core 200 and the toe 300.

Although not shown in FIGS. 1-7, it should be understood that a pylon may be coupled to the talus body 100, for example, when the artificial foot 10 is configured to serve as a prosthesis. As discussed herein, the artificial foot 10 may provide a more natural walking motion for the user because of the joints 12, 14 operatively coupling the talus body 100, the core 200 and the toe 300. Details of specific embodiments of the talus body 100, the core 200 and the toe 300 are discussed below.

The talus body 100 may be described as including a frame structure 110 and a front section 120, extending generally in a direction of the toe 300 when the artificial foot 10 is assembled. The talus body 100 may be made of a suitably strong, structural material, such as stainless steel. The talus body 100 may be machined, for example, with various cutouts 102 for weight savings. Alternatively, the talus body 100 may be cast, for example, using a suitable steel.

An upper portion 112 of the frame structure 110 of the talus body 100 may include a coupling or engagement means 114 configured to couple/engage with a pylon, for example, as in the case of the artificial foot being implemented as a prosthesis. The coupling/engagement means 114 may be in the form of a well-known "pyramid connector" and may be formed as an integral part of the frame structure 110, as appropriate or desired, for example, whether by machining or casting.

A mid-portion 116 of the frame structure 110 may provide a substantially planar upper bearing surface 116a and may include an aperture 116b therein. As described further below, the upper bearing surface 116a and the aperture 116b are employed to movably couple the talus body 100 with the core 200.

A lower portion 118 of the frame structure 110 may provide a substantially planar lower bearing surface 118a and may include a pair of apertures 118b therein. As described further below, the lower bearing surface 118a and the apertures 118b are employed to movably couple the talus body 100 with the toe 300.

The front section 120 of the talus body 100 may generally comprise a shaped vertical column. The front section 120 may include a relatively thicker (laterally) front end 122 with shaped side bearing surfaces 122a that taper away from a thicker central portion, as described in more detail below. As described further below, the bearing surfaces 122a of the front end 122 are engaged and constrained by corresponding surfaces of the core 200 during operation of the artificial foot 10.

The front section 120 of the talus body 100 may further include a reinforced aperture or bearing 124 for retaining a lateral axle 124a. In some embodiments, the lateral axle 124a may be formed integrally with the talus body 100, eliminating any need for an aperture or bearing.

One or more bumpers 130 may be mounted on a bottom surface or surfaces 132 of the frame structure 110 of the talus body 100. Such bumpers 130 may be made of urethane, for example, and may serve to cushion and/or limit downward movement of the talus body 100 during operation of the artificial foot 10. Alternatively, the bumper(s) 130 may be mounted on the core 200, as appropriate or desired.

The core 200 may include a heel-strike portion 210, a mid-foot bearing portion 220 and a toe engagement portion 230. These portions may be formed as an integral structure, for example, by injection molding a high performance plastics material, such as polyetheretherketone (PEEK), a bearing-grade plastic.

The heel strike portion 210 may extend rearwardly beneath the frame structure 110 of the talus body 100 when the artificial foot 10 is assembled, and may provide impact absorption for the artificial foot 10 in use. In embodiments, the heel strike portion 210 may include upwardly extending side walls 212 that may increase the strength and resilience of the heel strike portion 210. In embodiments, the side walls 212 may extend substantially to a rear edge 214 of the heel strike portion 210.

It should be noted that the outer surfaces of the core 200 may be suitably shaped to facilitate fitting the artificial foot 10 into shoes or other prosthetic devices. In particular, the outer dimensions of the core 200 may be symmetrical about a longitudinal axis (heel-to-toe) of the artificial foot 10. Thus, the core 200 may be a universal device, serving as either a right or left foot as desired.

The heel strike portion 210 and/or the mid-foot bearing portion 220 may include a retaining means 216, such as a recess and aperture as shown, configured to receive and retain one end of a tension/limit member, as described further below, for movably coupling the talus body 100 with the core 200.

The mid-foot bearing portion 220 may include a central channel or cavity 222. The cavity 222 may open rearwardly and upwardly, and may define side bearing surfaces 222a. The side bearing surfaces 222a may be shaped or otherwise configured to cooperate with the side bearing surfaces 122a of the front end 122 of the talus body 100 to constrain relative motion of the talus body 100 and the core during operation of the artificial foot 10.

The mid-foot bearing portion 220 may also include a pair of bearing recesses 224 defined in opposite inner walls of the central channel or cavity 222. The bearing recesses 224 may open rearwardly and may be configured to receive and retain respective bearings 226, each of which is configured to receive and retain an opposite end of the lateral axle 124a of the front section 120 of the talus body 100 when the artificial foot 10 is assembled. The bearing recesses 224 may be tapered to narrow in a forward direction, that is, toward the toe engagement portion 230. The bearings 226 may be similarly tapered to fit within the respective bearing recess 224. The lateral axle 124a may have a suitable degree of play within the bearings 226, for example, to avoid friction and heat. Further, the bearing recesses 224 may allow for a desired degree of movement of the respective bearings 226 within the bearing recesses 224. For example, a desired degree of play should exist to allow the interaction of the side bearing surfaces 122a of the front end 122 of the talus body 100 with the side bearing surfaces 222a of the cavity 222 of the mid-foot bearing portion 220 of the core 200 to govern a desired range of motion between the talus body 100 and the core 200 during operation of the artificial foot 10. When coupled, the lateral axle 124a, bearings 226 and bearing recesses 224 thus may allow constrained relative rotation and translation between the talus body 100 and the core 200.

It should be understood that, in some embodiments, the bearings 226 may be omitted and that the bearing recesses 224 may be configured to serve as bearings themselves. In such case, the bearing recesses 224 need only be configured to cooperate with the lateral axle 124a of the talus body 100 to provide the desired limited relative movement.

The mid-foot bearing portion 220 may also include a pair of generally longitudinal cannels 228, each of which opens to the heel-strike portion 210 and to the toe engagement portion 230. As described further below, the channels 228 may be configured to receive respective tension/limit members to couple the talus body 100 with the toe 300.

The toe engagement portion 230 may generally comprise a narrowed section or protrusion relative to the mid-foot bearing portion 220. As described further below, the toe engagement portion 230 may include curved or rounded upper and lower front edges to accommodate relative rotation of the toe 300, to provide a weight bearing surface during use, and to provide a smooth contact surface for an upper tension/limit member of the toe 300.

An upper portion of the toe engagement portion 230 and/or the mid-foot bearing portion 220 may include a retaining means 232, such as the recess and groove shown, configured to receive and retain an end of a plantarflexion tension/limit member 240 configured to constrain rotation of the toe 300 relative to the core 200.

A front end face 234 of the toe engagement portion 230 may include an upper pair of recesses 236 and a lower pair of recesses 238. Each of the recesses 236, 238 may be generally spherical and open forwardly. Each of the recesses 236, 238 may further include a narrower lateral portion that opens to a respective side of the toe engagement portion 230. As such, each of the recesses 236, 238 is configured to receive and movably retain one end of a respective link 250. In particular, the links 250 may be held in the recesses 236, 238 by a suitable mechanical assembly 234c, such as a washer and fastener as illustrated.

Each of the links 250 may comprise a metal, such as stainless steel, for strength and wear resistance. Each link 250 may be a unitary member in a generally "dumbbell" configuration, with spherical end portions interconnected by a narrower, centralized cylindrical portion. It should be understood that the links 250 need not be identical, nor symmetrical, although shown as such. Some or all of the links 250 may also be cable, with spherical swages on each end.

The toe 300 may comprise a generally U-shaped rear bracket portion 310 and a tapered, forwardly extending plantar portion 320. A rear end face 312 of the bracket portion 310 may include an upper pair of recesses 314 and a lower pair of recesses 316. Each of the recesses 314, 316 may be generally hemispherical and open rearwardly. Each of the recesses 314, 316 may further include a narrower lateral portion that opens to a respective side of the toe 300.

The rear bracket portion 310 may be configured to receive the toe engagement portion 230 at least partially therein when the artificial foot is assembled. As each of the recesses 314, 316 is configured to receive and movably retain the other end of a respective link 250 that is engaged by the respective recess 236, 238 of the toe engagement portion 230, the links 250 movably couple the toe engagement portion 230 of the core 200 with the toe 300 to allow the toe 300 to rotate generally about a lateral axis during use of the artificial foot 10. Using pairs of links 250, an overrange of rotation of the toe 300 relative to the core 200 may be possible. For example, the links 250 in the upper recesses 236, 314 may remain engaged while the links 250 in the lower recesses 238, 316 partially disengage, and vice versa, as appropriate or desired, with other tension/limit members maintaining joint integrity.

The rear bracket portion 310 may include a pair of retaining means 318, such as recesses and bores as shown, on opposite sides thereof, each of which is configured to receive and retain an end of a tension/limit member 260 configured to constrain relative movement of the toe 300 and the talus body 100, as described further below.

The plantar portion 320 may also include a retaining means 322, such as a recess and bore or aperture as shown, configured to receive and retain the other end of the plantarflexion tension/limit member 240 configured to constrain rotation of the toe 300 relative to the core 200.

Returning to the tension/limit members 260 coupling the toe 300 with the talus body 100 when the artificial foot is assembled, each of the members 260 extends through a respective one of the longitudinal cannels 228 in the mid-foot bearing portion 220 of the core 200, and through a respective one of the apertures 118b in the lower bearing surface 118a of the lower portion 118 of the frame structure 110 of the talus body 100.

An opposite end 262 of each of the tension/limit members 260 may be retained by a toe spring carrier assembly 270 disposed adjacent the lower bearing surface 118a of the lower portion 118 of the frame structure 110 of the talus body 100. The opposite ends 262 may be retained in any suitable manner, for example, by a recess and aperture formed in a closed end 272 of an encasement 274. Each of the ends of the tension/limit members 260 may be secured using a respective spherical bushing 264 (FIG. 7) to provide a bearing surface for smooth operation of the artificial foot 10.

The encasement 274 may define respective cavities 276 with an open end opposite the closed end 272, each of the cavities 276 being configured to receive and retain a respective elastomeric members 278 through which the respective tension/limit members 260 extend axially. Additionally or alternatively, the cavities 276 may receive and retain coil springs. In a relaxed, or substantially uncompressed state, the elastomeric members 278 (and/or coil springs) extend beyond the encasement 274. During use, when the toe 300 is rotated upwardly the tension/limit members 260 will be tensioned and pull encasement 274 of the toe spring carrier assembly 270 toward the lower bearing surface 118a, compressing the elastomeric members 278 (and/or coil springs). Engagement of the encasement 274 with the lower bearing surface 118a provides a stop limit through the tension/limit members 260 for the upward rotation of the toe 300. Further, because the elastomeric members 278 are compressed during such rotation of the toe 300, whether or not the stop limit is reached, the elastomeric members 278 may provide actuation for downward rotation of the toe 300 through the tension/limit members 260, for example, during toe-off of a stepping movement with the artificial foot 10. Also during use, the elastomeric members 278 (and/or coil springs) may be compressed by movement of the talus body 100, the "pre-loading" the toe 300. This may enhance stability and store additional energy to be released during toe-off.

Returning to the movable coupling of the talus body 100 with the core 200 via the upper bearing surface 116a and the aperture 116b, a tension/limit member 280 may have one end engaged by the retaining means 216 and may extend through the aperture 116b, with an opposite end 282 engaged by a core spring carrier assembly 290 disposed above the upper bearing surface 116a of the upper portion 116 of the frame structure 110 of the talus body 100. The opposite end 282 may be retained in any suitable manner, for example, by a recess and aperture formed in a closed end 292 of an encasement 294. In the embodiment shown, the tension/limit member 280 may be a solid rod, for example, made of steel or other suitable metal, which may include threaded ends for securing to the retaining means 216 and/or the encasement 294. In particular, each of the ends of the tension/limit member 280 may be secured using a respective bushing 282 (FIG. 7) to provide a bearing surface for smooth operation of the artificial foot 10.

In the embodiment illustrated, the use of a solid rod for the tension/limit member 280 may provide particular advantages. For example, fatigue strength may be improved, or at least defined, for a rod as compared to a rope for this use. Further, cost may be reduced and ease of assembly may be facilitated using a rod. For example, the rod may comprise a socket head cap screw.

The encasement 294 may define a plurality of cavities 296, each with an open end opposite the closed end 292, each of the cavities 296 being configured to receive and retain a respective coil spring 298 axially oriented generally parallel to the tension/limit member 280. Additionally or alternatively, the cavities 296 may receive and retain an elastomeric material. In a relaxed, or substantially uncompressed state, the coil springs 298 (and/or elastomeric material) extend beyond the encasement 294. During use, when the talus body 100 is rotated forwardly relative to the core 200 the tension/limit member 280 will pull encasement 294 of the spring carrier assembly 290 toward the upper bearing surface 116a, compressing the coil springs 298 (and/or elastomeric material). Engagement of the encasement 294 with the upper bearing surface 116a provides a stop limit through the tension/limit member 280 for the forward rotation (e.g., pitch) of the talus body 100. Further, because the coil springs 298 are compressed during such rotation of the talus body 100, whether or not the stop limit is reached, the coil springs 298 may provide actuation for downward rotation of the core 200 through the tension/limit member 280, for example, during a forward stepping movement with the artificial foot 10.

It should be understood that the constraints provided by the various tension/limit members may not only influence motion of the joints 12 and 14, but also may stabilize the joints 12 and 14. Moreover, as appropriate or desired, the tension ropes may be configured to store and release energy during walking movements of the artificial foot 10, thereby mimicking performance characteristics of the human foot.

By employing grooves for the tension/limit members, particularly tension/limit ropes, the members may be maintained in proper place and may be less likely to be damaged. Although not specifically depicted in the drawings for the sake of simplicity, it should be understood that means for adjusting the tension of the various tension ropes may be included in a practical implementation. Any suitable adjustment mechanism may be employed.

The bottom of the core 200, and the bottom of the toe 300 to some degree, may serve as load-bearing surfaces during walking movements of the artificial foot 10. The loads may be transferred through the joints 12 and 14 to provide a more realistic walking performance of the artificial foot 10.

It should be noted that for each of the tension members secured to a respective member by a ball-end, as illustrated in some of the figures, a bushing made of a suitable material, such as a plastics material, may be included to provide a suitable surface for movement of the ball relative to the respective member. This may facilitate smooth movement of the corresponding ropes and/or prevent wear at the rope connections.

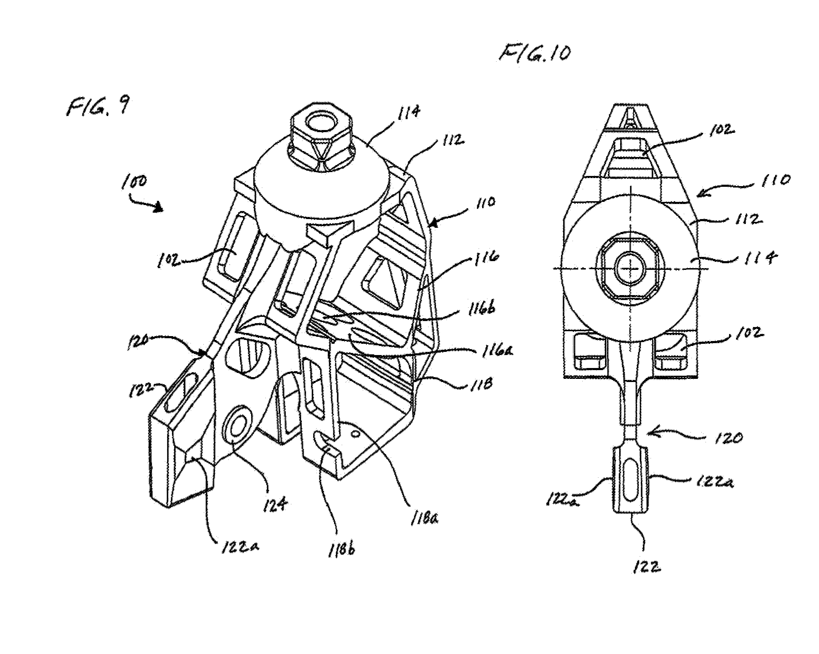

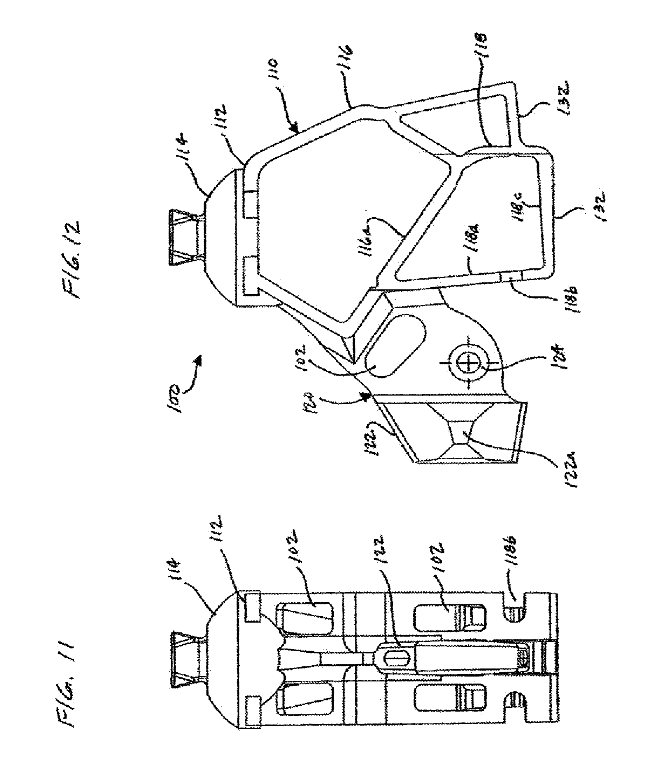

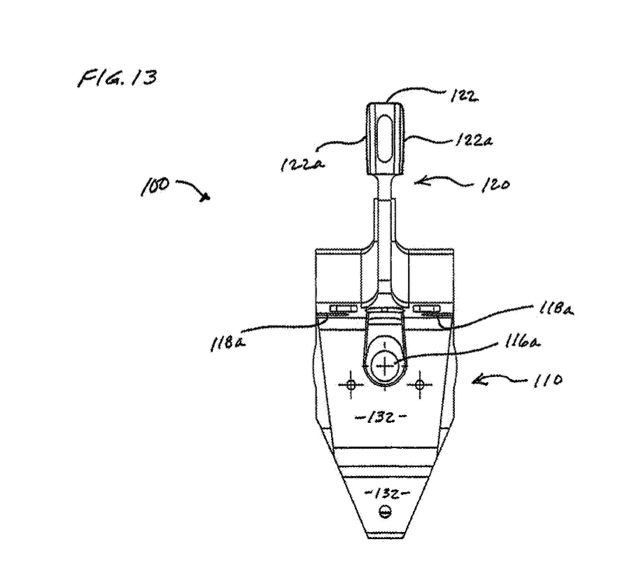

Turning to FIGS. 9-13 of the talus body 100 in isolation, various features described above are illustrated without obstruction. The frame structure 110 including the upper portion 112, the mid-portion 116, the lower portion 118 and cutouts 102 is shown. Again, the coupling or engagement means 114 may be implemented as a well-known "pyramid connector."

The mid-portion 116 of the frame structure 110 provides the substantially planar upper bearing surface 116a and includes the aperture 116b, which are employed to movably couple the talus body 100 with the core 200 via the tension/limit member 280 and the core spring carrier assembly 290. The lower portion 118 of the frame structure 110 provides the substantially planar lower bearing surface 118a and include the apertures 118b, which are employed to movably couple the talus body 100 with the toe 300 via the tension/limit member 260 and the toe spring carrier assembly 270.

It should be noted that the relative angle of the upper bearing surface 116a may be configured such that the maximum loading of the tension/limit member coupling the talus body and the core occurs when the longitudinal axis of that tension/limit member is substantially perpendicular to the plane of the upper bearing surface 116a. This configuration may help to maintain direct compression of the coil springs (and/or elastomeric material) and limit translation of the coil springs (and/or elastomeric material) along the upper bearing surface 116a.

Similarly, it should be noted that the relative angle of the lower bearing surface 118a may be configured such that when the mid-foot joint and the toe joint are at a maximum range of motion, the plane of the lower bearing surface 118a is substantially perpendicular to the longitudinal axis of the tension/limit member or the longitudinal axis that runs from the toe. This configuration may help to maintain direct compression of the elastomeric members (and/or coil springs) and limit upward/downward movement of the elastomeric members (and/or coil springs) along the lower bearing surface 118a. Also, a bottom wall surface 118c of the lower portion 118, upon which the encasement of the toe spring carrier assembly may movably rest, may be disposed at a relatively small angle (such as 2 or 3 degrees) relative to the lower bearing surface 118a, for example, to accommodate for a corresponding degree of draft that may be employed when the encasement is made by an injection molding process.

The front section 120 including the front end 122 with shaped side bearing surfaces 122a may be understood particularly from the talus body 100 shown in isolation. As discussed herein, the bearing surfaces 122a of the front end 122 are engaged and constrained by corresponding surfaces of the core 200 during operation of the artificial foot 10. In general, the front and rear of the bearing surfaces 122a may be configured to limit rotation of the core 200 relative to the talus body 100 about a substantially vertical axis, substantially parallel to the pylon for example. The upper and lower of the bearing surfaces 122a may be configured to limit rotation (e.g., twisting) of the core 200 relative to the talus body 100 about a substantially horizontal axis, substantially in-line with the talus-core-toe assembly. The particular angles of the bearing surfaces 122a and the distances between the bearing surfaces 122a and the corresponding bearing surfaces 222a in the cavity 222 of the core 200 may be critical to the design as such parameters may control the amount of relative motion that is allowed between the talus body 100 and the core 200, in addition to the limits placed on rotation about the lateral axle 124a. The bearing surfaces may be designed to contact corresponding bearing surfaces with maximum amounts of surface area during use to decrease stress of the surfaces and increase surface longevity.

It should be noted that the opposing upper/lower bearing surfaces 122a may not be entirely parallel so as to correspond and cooperate with the respective bearing surfaces 222a of the core 200 when the core 200 is made by an injection molding process. Specifically, the upper/lower bearing surfaces 122a may be 2 to 3 degrees from parallel to account for the 2 to 3 degree draft angle of the injection molding process. This may also be true for the opposing front/rear bearing surfaces 122a.

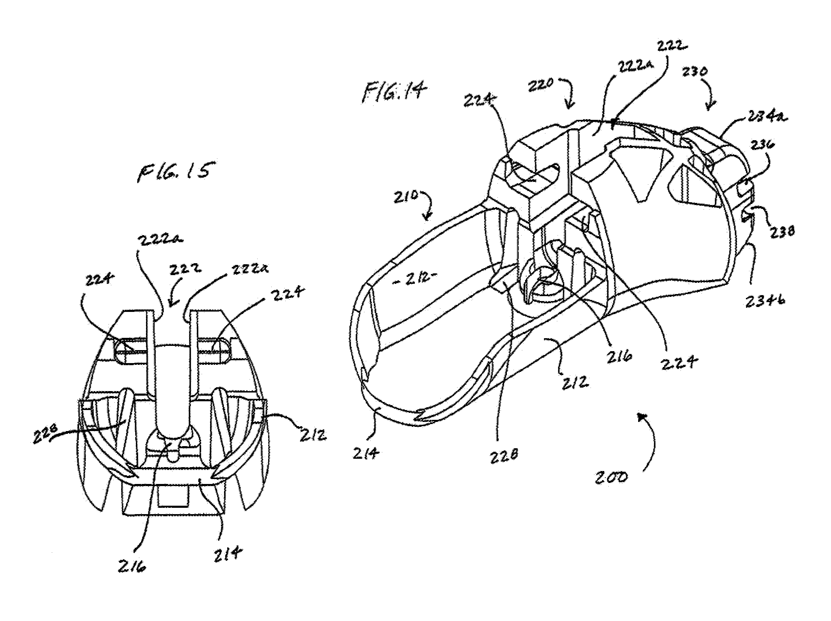

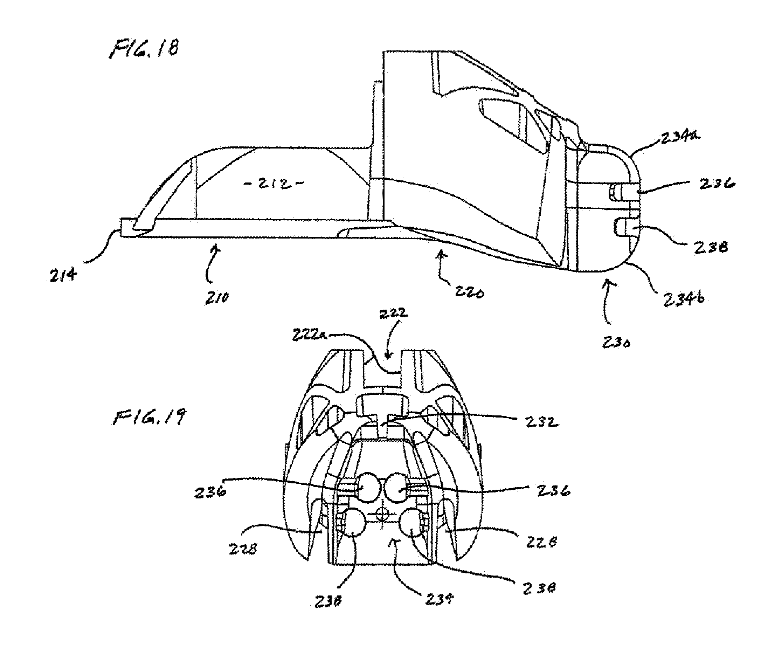

Turning to FIGS. 14-19 of the core 200 in isolation, various features described above are illustrated without obstruction. As described above, the core 200 includes the heel-strike portion 210, the mid-foot bearing portion 220 and the toe engagement portion 230. The heel strike portion 210 includes upwardly extending side walls 212 that may increase the strength and resilience of the heel strike portion 210. In embodiments, the side walls 212 may extend substantially to the rear edge 214 of the heel strike portion 210.

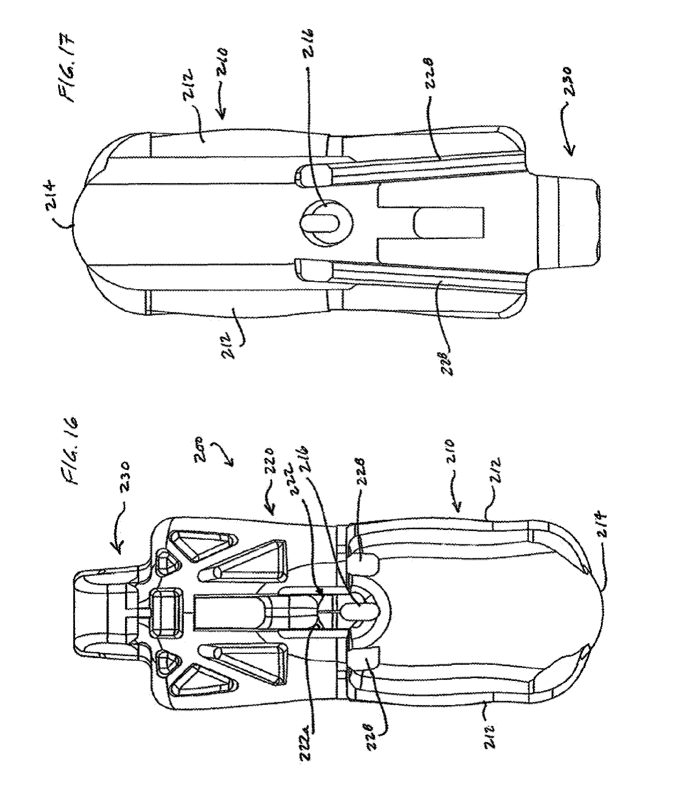

As particularly illustrated in FIGS. 16 and 17, the retaining means 216, such as the recess and aperture shown, configured for receiving and retaining one end of the tension/limit member movably coupling the core 200 with the talus body 100 opens to the bottom surface of the core 200 for ease of assembly. Also in these figures, the longitudinal channels 228 configured to receive respective tension/limit members to couple the talus body 100 with the toe 300 open to the heel-strike portion 210, on top, and to the toe engagement portion 230, on bottom.

The mid-foot bearing portion 220 includes the rearwardly opening cavity 222 with side bearing surfaces 222a. The mid-foot bearing portion 220 also includes the rearwardly opening bearing recesses 224 in opposite inner walls of the cavity 222 for receiving and movably retaining the respective bearings 226 when the artificial foot 10 is assembled. As discussed above, the bearing recesses 224 may be tapered to narrow in the forward direction, and may allow for a desired degree of movement of the respective bearings 226 within the bearing recesses 224. In the embodiment shown in these figures, there is only enough room for the axle to be received, not the bearings. In this embodiment, the core may be made of a suitable bearing material, so that additional bearings may not be required.

In the embodiment shown, the toe engagement portion 230 includes a curved or rounded upper front edge 234a and a curved or rounded lower front edge 234b. In particular, the upper and lower front edges 234a, 234b may accommodate relative rotation of the toe 300 about the front end face 234 of the core 200. The rounded lower front edge 234b may provide a weight bearing surface during use, allowing a "rolling contact" surface during heel lift-off. The rounded upper front edge 234a may provide a smooth contact surface for the upper plantarflexion tension/limit member 240 of the toe 300.

As described above, the upper portion of the toe engagement portion 230 and/or the mid-foot bearing portion 220 may include the retaining means 232, such as the recess and groove shown, configured to receive and retain an end of a plantarflexion tension/limit member 240 configured to constrain rotation of the toe 300 relative to the core 200.

Further, the front end face 234 of the toe engagement portion 230 includes the recesses 236, 238 configured to receive and movably retain one end of respective links 250. In particular, the links 250 may be held in the recesses 236, 238 by a suitable mechanical assembly (not shown) or by use of adhesives.

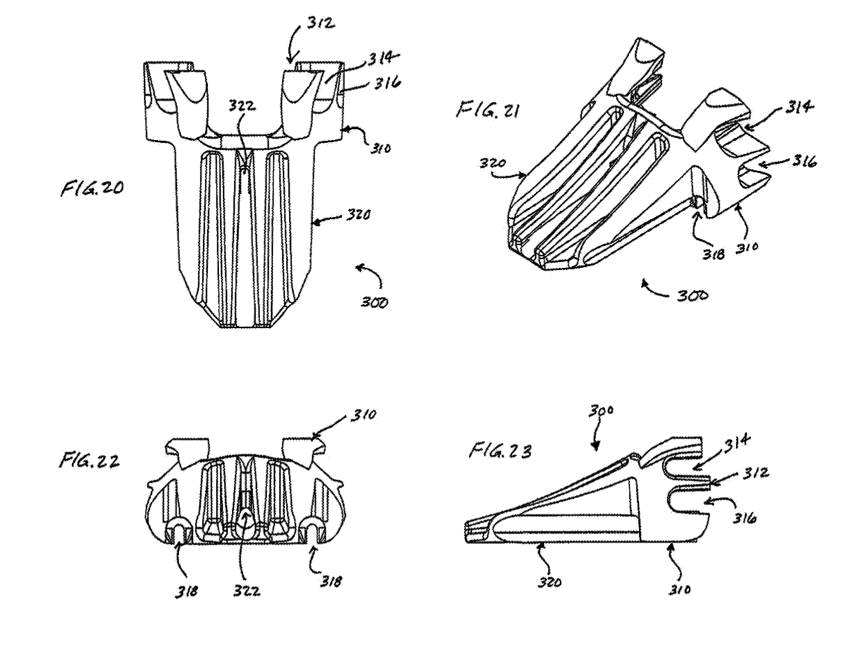

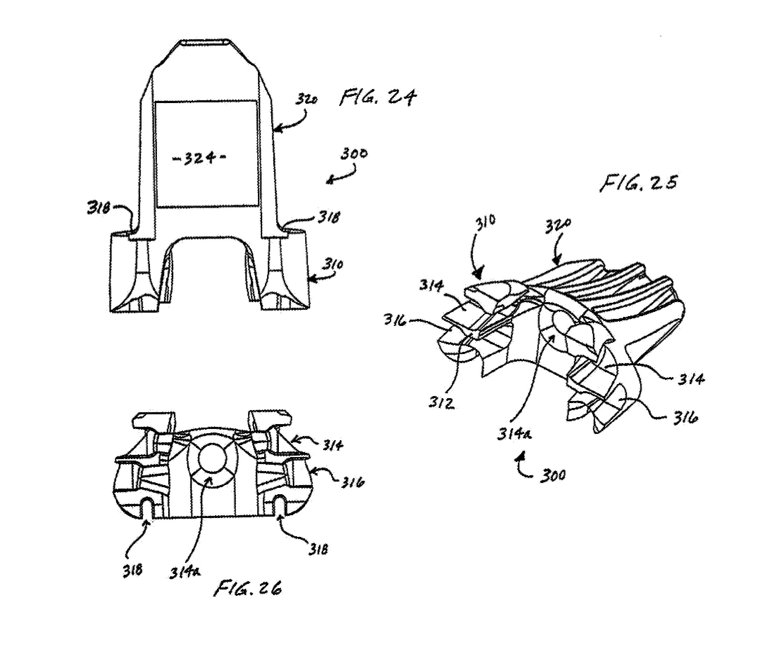

Turning to FIGS. 20-26 of the toe 300 in isolation, various features described above are illustrated without obstruction. As described above, the toe 300 includes the rear bracket portion 310 and the forwardly extending plantar portion 320. The rear end face 312 of the bracket portion 310 includes the upper recesses 314 and the lower recesses 316 for receiving and movably retaining the other end of a respective link 250 that is engaged by the respective recess 236, 238 of the toe engagement portion 230 when the artificial foot 10 is assembled, with the links 250 movably coupling the toe engagement portion 230 of the core 200 with the toe 300 to allow the toe 300 to rotate generally about a lateral axis during use of the artificial foot 10.

The rear bracket portion 310 also includes the retaining means 318 configured to receive and retain ends of the respective tension/limit members 260. Also, the rear bracket portion 310 includes a recess or relief 314a for accommodating the mechanical assembly used to hold the links 250 in the recesses 236, 238 on the front end face 234 of the toe engagement portion 230.

The plantar portion 320 includes the retaining means 322 configured to receive and retain the other end of the plantarflexion tension/limit member 240 configured to constrain rotation of the toe 300 relative to the core 200. As particularly shown in FIG. 22, for example, the retaining means may comprise an open cavity in an upper portion of the plantar portion 320 that opens upwardly through a relatively narrower open channel. Thus, the plantarflexion tension/limit member 240 may extend through the channel with its end secured in the cavity of the retaining means 322. It should be noted that the retaining means 322 does not extend through to a bottom plantar surface 324, to avoid any potential

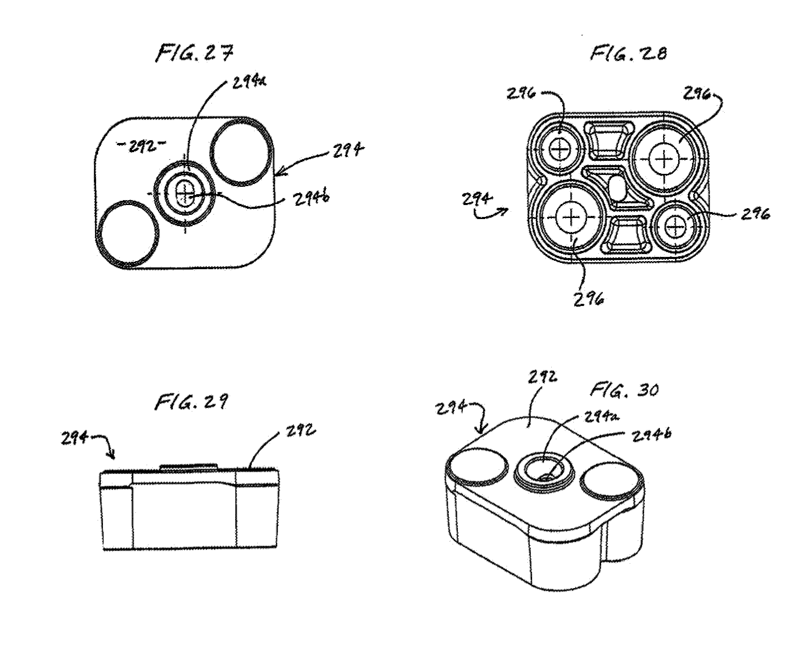

Turning to FIGS. 27-30 of the encasement 294 of the core spring carrier assembly 290 in isolation, various features described above are illustrated without obstruction. As described above, the tension/limit member 280 may have one end engaged by the retaining means 216 and may extend through the aperture 116b of the frame structure 110, with an opposite end 282 engaged/retained in a suitable manner, such as by a recess 294a and aperture 294b formed in the closed end 292 of the encasement 294.

As illustrated, the encasement 294 defines a plurality of cavities 296, each with an open end opposite the closed end 292, each of the cavities 296 being configured to receive and retain a respective coil spring, elastomeric member and/or elastomeric material, as described above. It should be understood that the cavities 296 may be of the same size, either depth and/or width, to accommodate corresponding elements as desired.

Further, it should be understood that the cavities 296 may be of different sizes, either depth and/or width, to accommodate differing corresponding elements as desired. It may be desirable to have one or more elements loaded in succession (variable timing of loading/actuation) by having the elements extend different lengths from the encasement 294. This may be achieved by varying the lengths of the elements, the depths of the cavities, or both. Similarly, different loading characteristics may be achieved by varying the length and/or width of the elements and the corresponding cavity dimensions.

For example, the cavities 296 may be configured to hold the elements at different depths such that not all elements begin to be compressed by the motion of the mid-foot joint. This action directly impacts the torque response curve of the mid-foot joint. If the larger elements (e.g., springs) contact the bearing surface first, there is a larger initial stiffness of the joint. If the smaller elements contact the bearing surface first, there is a smaller initial stiffness of the joint. Thus, the length of the elements may be used to "tune" the torque response curve.

In the embodiment shown, even if different sized cavities 296 are employed, the cavities 296 may be disposed symmetrically relative to the longitudinal (heel-to-toe) axis and/or the lateral axis of the artificial foot 10 to provide a neutral response.

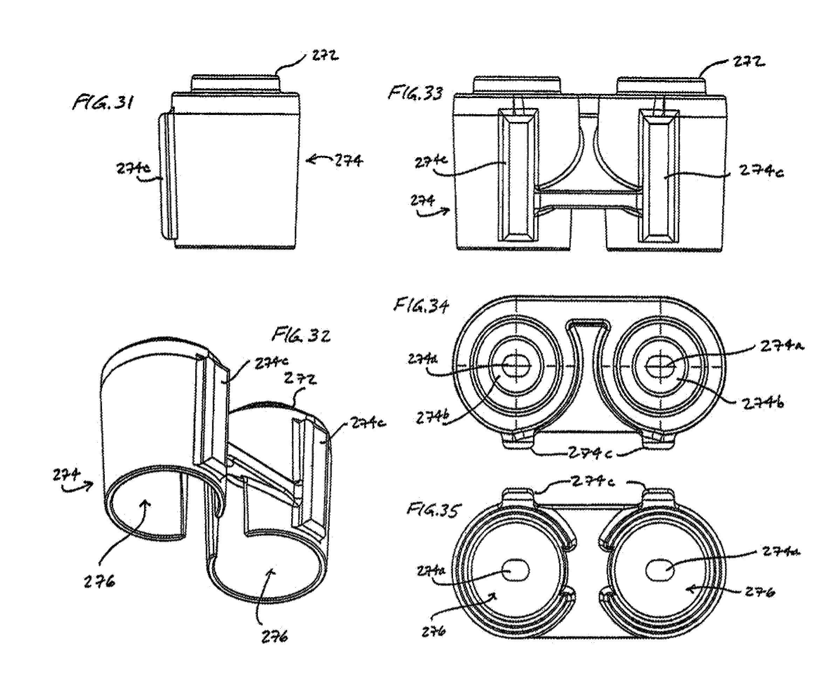

Turning to FIGS. 31-35 of the encasement 274 of the toe spring carrier assembly 270 in isolation, various features described above are illustrated without obstruction. As described above, each of the tension/limit members 260 may have one end engaged by the respective retaining means 232 and may extend through the respective aperture 118b of the frame structure 110, with an opposite end 262 engaged/retained in a suitable manner, such as by a recess 274a and aperture 274b formed in the closed end 272 of the encasement 274.

As illustrated, the encasement 274 defines a plurality of cavities 276, each with an open end opposite the closed end 272, each of the cavities 276 being configured to receive and retain a respective coil spring, elastomeric member and/or elastomeric material, as described above. It should be understood that the cavities 276 may be of the same size, either depth and/or width, to accommodate corresponding elements as desired. Further, it should be understood that the cavities 276 may be of different sizes, either depth and/or width, to accommodate differing corresponding elements as desired.

As discussed above, it may be desirable to have one or more elements loaded in succession (variable timing of loading/actuation) by having the elements extend different lengths from the encasement 274. In the embodiment shown, varying the relative performance of the elements disposed in the cavities 276 will vary the relative performance of the right and left tension/limit members 260, thus providing a non-linear/off-axis bias to the operation of the artificial foot 10. In other words, differences in the cavities 276 and/or differences in the elements (springs, elastomeric members) may provide a biased response, for example, to define a right or left prosthetic foot, or for other reasons. Such left/right differences may also be achieved by different properties of the respective tension/limit members 260, such as having different relative lengths, so that one side of the toe 300 has a greater range of motion than the other side.

The encasement 274 may further include raised surfaces, projections or rails 274c as illustrated. The rails 274c may increase wear resistance of the encasement 274 for its sliding movement against the bottom wall surface 118c. The rails 274c may also accommodate a 2 to 3 degree draft angle that the encasement 274 may have when made by an injection molding process. Further, the rails 274c may be set back from the open end of the encasement to avoid any potential interference with a filet or rounded corner in the frame structure 110 between the bearing surface 118a and the bottom wall surface 118c.

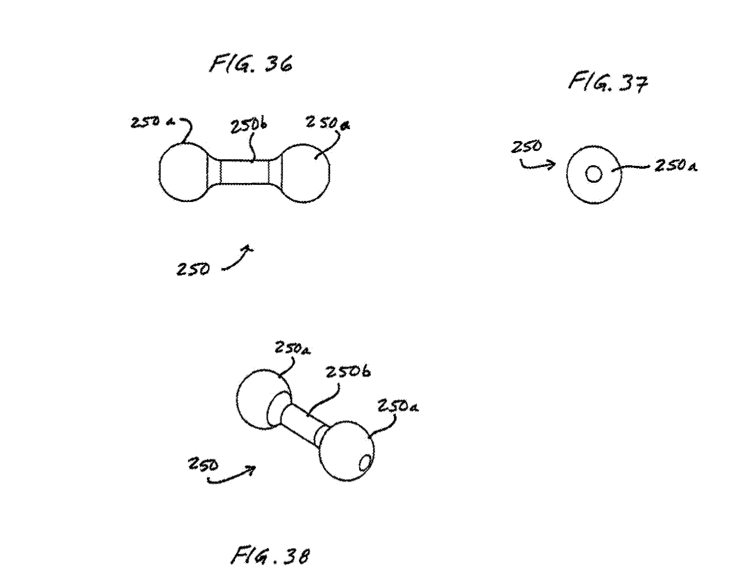

Turning to FIGS. 36-38 of the link 250 configured to movably couple the core 200 and the toe 300, various features described above are illustrated without obstruction. As described above, each link 250 may be a unitary member in a generally "dumbbell" configuration. The link 250 may have spherical end portions 250a interconnected by a narrower, centralized cylindrical portion 250b. It should be understood that other configurations of the links 250 are possible as well, although the curved surfaces of the "dumbbell" configuration may provide less potential for fatigue and/or failure of the parts of the artificial foot 10 contacted by the links 250.

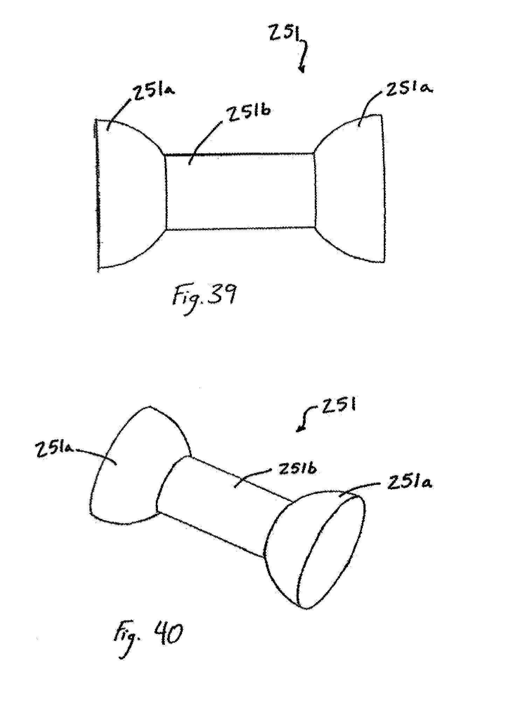

Turning to FIGS. 39-40, another link 251 provides an alternative to the link 250. Link 251 is also configured to movably couple the core 200 and the toe 300. Each link 251 may be a unitary member having semi-spherical ends with the planar ends of the partial sphere forming the terminal ends of each link 251. The semi-spherical end portions 251a may be interconnected by a narrower, centralized cylindrical portion 251b. The links 251 provide a modified "dumbbell" configuration at the ends, and include partial spheres with rounded ends that interconnect with the cylindrical portion 251b. As these spherical ends of the semi-spherical end portions 251a contact the core 200 and the toe 300, the spherical ends provide less potential for failure of the parts of the artificial foot 10 by spreading pressure over a larger surface area of the partial spherical end portions 251a.

Operation of the artificial foot 10 during walking movement may be described as follows. Beginning with heel strike, the heel strike portion 210 of the core 200 may absorb impact. The talus body 100 may move downward toward the heel strike portion 210 with the bumpers 130 (when provided) contacting the upper surface of the heel strike portion 210 to absorb the heel strike. The talus body 100 may rotate (counter-clockwise in FIG. 2) relative to the core 200 during downward movement of the talus body 100, moving the bearings 226 to a top of the bearing recesses 224 of the talus body core 200.

The talus body 100 may then rotate about a virtual lateral axis in an opposite direction (clockwise in FIG. 2) relative to the core 200 until the artificial foot reaches a neutral (standing support) position. In the neutral position, the bearings 226 may return to a bottom of the bearing recesses 224. Further in this position, the artificial foot may be supported via the bottom of the core 200 as a contact/support/load-bearing surface.

From the neutral position, tibial progression in a forward direction causes the talus body 100 to rotate about the axle 124a of the first joint 12, via the bearings 224 when present. The coil springs 298 in the spring carrier assembly 290 are loaded against the upper bearing surface 116a and the coil springs 278 in the spring carrier assembly 270 are loaded against the lower bearing surface 118a as the tension/limit member 260 is loaded.

Continued tibial progression forward causes heel rise or lift-off as well as rotation of the toe 300 relative to the core 200. As the heel rises, the artificial foot 10 is supported by the toe engagement portion 230 of the core 200, with the toe 300 continuing to rotate. Such movement may occur until the limits of the tension/limit members 240, 260, 280 are reached.

In this embodiment, the tension/limit members 240 and 260 may have some elastic properties and may be fabricated of, for example, aramid fiber rope, stainless steel cable, galvanized cable, or Nitinol. Thus, the tension/limit members 240, 260 may be stretched by the rotation of the toe 300. One example of an elastic material for the tension/limit members 240, 260 is Nitinol. In such case, the stretching will be a maximum at the phase change for Nitinol. Once the limit(s) of the tension/limit member(s) 240, 260 has/have been reached, the artificial foot 10 may push off with the toe 300. In particular, the toe 300 may push off in a forward and upward direction, releasing energy stored in the tension/limit members 240, 260 in that direction as the artificial foot 10 is lifted by the user, in addition to the energy stored by the coil springs 278, 298 discussed above.

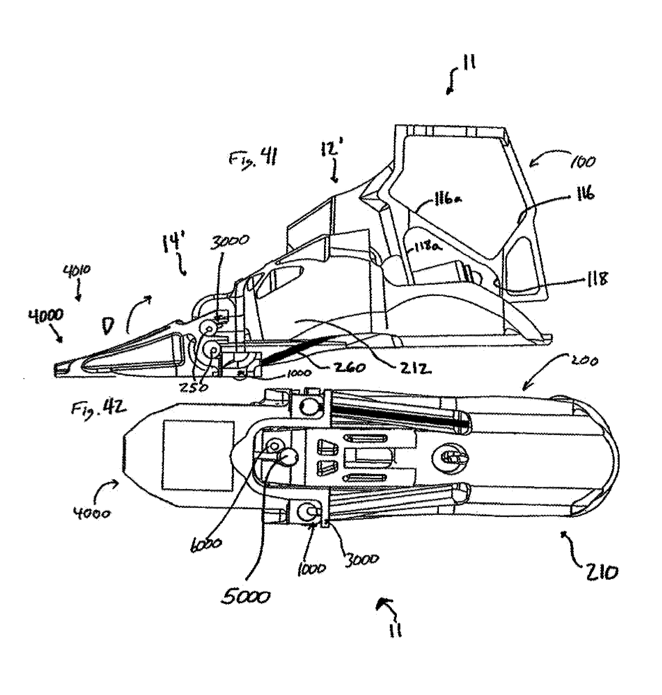

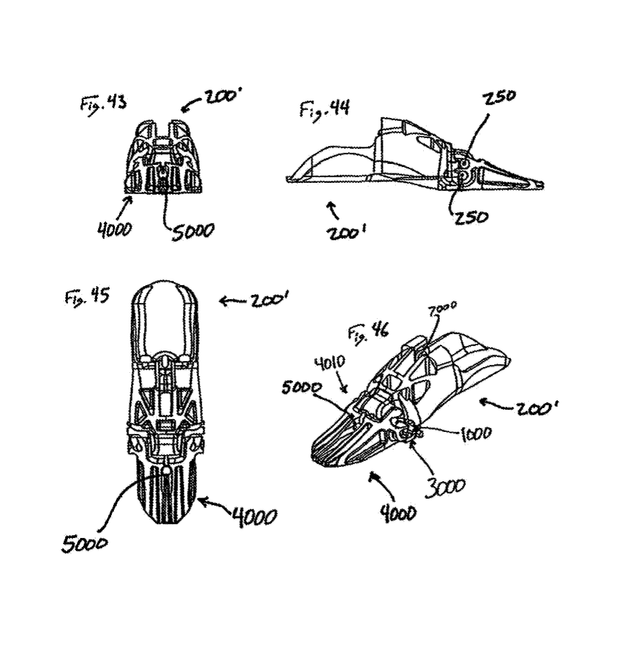

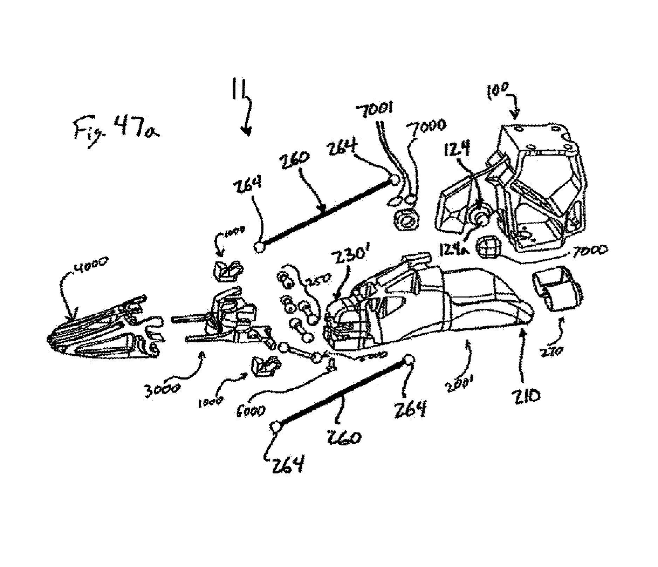

Turning to FIG. 41, another implementation of an artificial foot 11 is illustrated. The artificial foot 11 includes a talus body 100, a core 200', a toe 4000, first joint 12' and second joint 14'. FIG. 42 illustrates a bottom view of the artificial foot 11. FIG. 43 illustrates a front view of the core 200 and the toe 4000 of the artificial foot 11. FIG. 44 illustrates a right side view of the core 200 and the toe 4000 of the artificial foot 11. FIG. 45 illustrates a top view of the core 200 and the toe 4000 of the artificial foot 11. FIG. 46 illustrates an isometric view of the core 200 and the toe 4000 of the artificial foot 11. FIG. 47a illustrates an exploded view of the artificial foot 11.

The artificial foot 11 differs from the artificial foot 10 described above in connection with FIGS. 1-40. A toe assembly 4010 of the foot 11 includes a toe 4000, a toe insert 3000 and toe bushings 1000. The artificial foot 11 may not include a plantarflexion tension/limit member 240, but may constrain the motion of the toe 4000 by way of a vertical restraint link 5000 in which couples the toe 4000 and the core 200'. The artificial foot 11 also includes pivot bearings 7000 that may be configured to absorb forces generated by movement of the talus 100 relative to the core 200'

The talus body 100 of the artificial foot 11 is structurally and functionally the same as the talus body 100 of the artificial foot 10 described above. Other than the differing structures and functions described below in connection with the core 200' of the artificial foot 11, the core 200' is similar to the core 200 of the artificial foot 10 in certain other structural and functional respects. For example, the core 200' includes a heel-strike portion 210, and other than the features described herein, the mid-foot bearing portion 220' and toe engagement portion 230' of the core 200' are respectively similar to the mid-foot bearing portion 220 and the toe engagement portion 230 of the core 200 of the artificial foot 10. Other than the differing structures and functions described in connection with the toe 4000 of the artificial foot 11, the toe 4000 is similar to the toe 300 of the artificial foot 10 in other structural and functional respects.

Turning to FIG. 47a, the artificial foot 11 includes the talus body 100, the core 200', the links 250, tension/limit members 260, the toe spring carrier 270, bushings 1000, a toe insert 3000, a toe 4000, a vertical restraint link 5000, a retaining screw 6000, and pivot bearings 7000. The structure and function of the talus body 100, links 250, tension/limit members 260 and the toe spring carrier 270 of the artificial foot 11 are the same as the corresponding structures of the artificial foot 10. It will be understood that, while not shown in FIG. 47a, the talus body 100 includes the spring carrier assembly 290 and its related components such as tension/limit member 280. The structural and functional details of the core 200', bushings 1000, toe insert 3000, toe 4000, vertical restraint link 5000, retaining screw 6000, and pivot bearings 7000 are provided below.

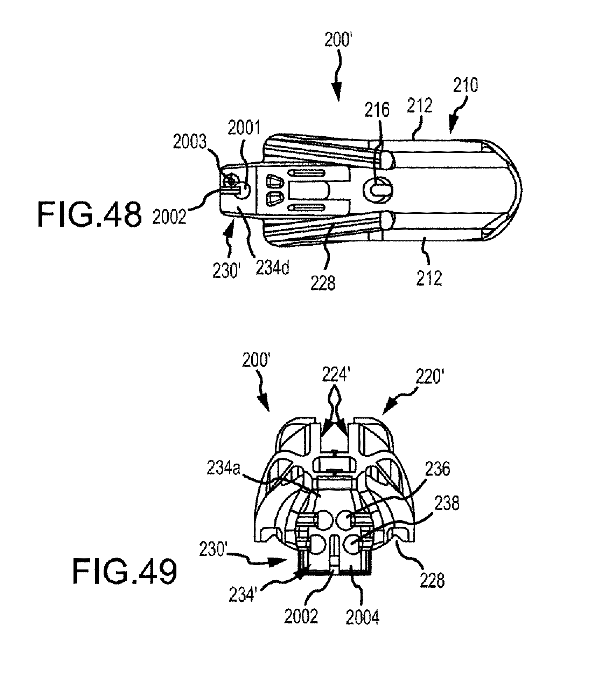

Turning to FIGS. 48-49 of the core 200' of the artificial foot 11 in isolation, various features are described without obstruction. FIG. 48 is a bottom view of the core 200'. FIG. 49 is a front view of the core 200'. The core 200' includes a modified toe engagement portion 230' with vertical restraint link ball holder 2001, a restraint link passage 2002, threaded walls 2003 defining a void for receiving a retention screw 6000 (FIG. 47a), and an external surface 2004 for contacting toe 4000.

The toe engagement portion 230' may generally comprise a narrowed section or protrusion relative to the mid-foot bearing portion 220'. In addition to lateral recesses 236, 238, a vertical restraint link ball holder 2001 and a restraint link passage 2002 are formed by walls of the toe engagement portion 230'. The vertical restraint link ball holder 2001 may form a recess at a bottom portion or rolling surface 234d of the core. The recess may be generally spherical and open at the rolling surface 234d of the toe engagement portion 230'. The restraint link passage 2002 includes a narrower portion that extends from the recess towards the front surface 234' of the toe engagement portion 230' where the passage 2002 forms an opening. As such, the ball holder 2001 and restraint link passage 2002 together are configured to receive and movably retain one end of the vertical restraint link 5000. The threaded walls 2003 defining the void are formed at the rolling surface 234d of the toe engagement portion 230' and taper to form an opening complementary to a retention screw 6000 as the walls extend up into the toe engagement portion 230'. The external surface 2004 of the toe engagement portion 230 may include curved or rounded upper and lower front edges to accommodate relative rotation of the toe 4000 and to provide a weight bearing surface during use.

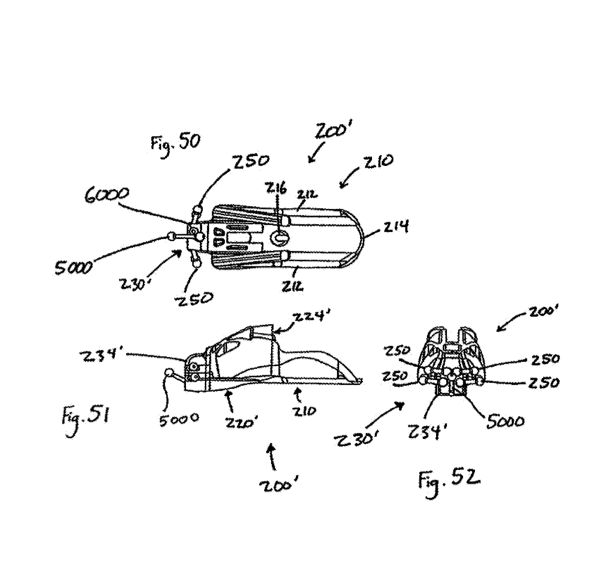

FIG. 50 illustrates a bottom view of the core 200' with the vertical restraint link 5000. Upon assembling the vertical restraint link 5000 in the toe engagement portion 230', the retention screw 6000 is engaged with the threaded walls 2003 in order to hold the vertical restraint link 5000 within the toe engagement portion 230' in a movably coupled conformation. Other mechanical means in addition or as an alternative to the retention screw 6000 may also be provided for retaining the vertical restraint link 5000 within the toe engagement portion 230' of the core 200'.

FIG. 51 illustrates a left side view of the core 200' with the vertical restraint link 5000 extending along the restraint link passage 2002 and through the front end face 234' of the toe engagement portion 230'.

FIG. 52 illustrates a front view of the core 200' with the vertical restraint link 5000 positioned in front of the four links 250. The vertical restraint link 5000 is positioned along a longitudinal axis of the core 200' and extends from the core 200' along the longitudinal axis at an angle, e.g., approximately 45 degrees. The links 250 within the lateral recesses 236, 238 are arranged laterally with respect to the longitudinal axis and the vertical restraint link 5000, and thus may also be referred to as lateral links 250.

The vertical restraint link 5000 may comprise a metal, such as stainless steel, for strength and wear resistance. The vertical restraint link 5000 be a unitary member in a generally "dumbbell" configuration, with spherical end portions interconnected by a narrower, centralized cylindrical portion. Compared to the lateral links 250, the vertical restraint link 5000 may have a relatively longer centralized narrower portion (e.g., narrower cylindrical shape, oval-shaped or with rounded and flattened portions). The spherical portions may alternatively be semi-spherical, as discussed above in connection with FIGS. 39-40. The vertical restraint link 5000 may also include a cable portion with spherical swages on each end. In connection with the artificial foot 11, the links 251 may also replace one or more of the lateral links 250. The links 5000, 250 and 251, may be fabricated of oil-impregnated bronze or oil-impregnated brass to provide for self-lubrication.

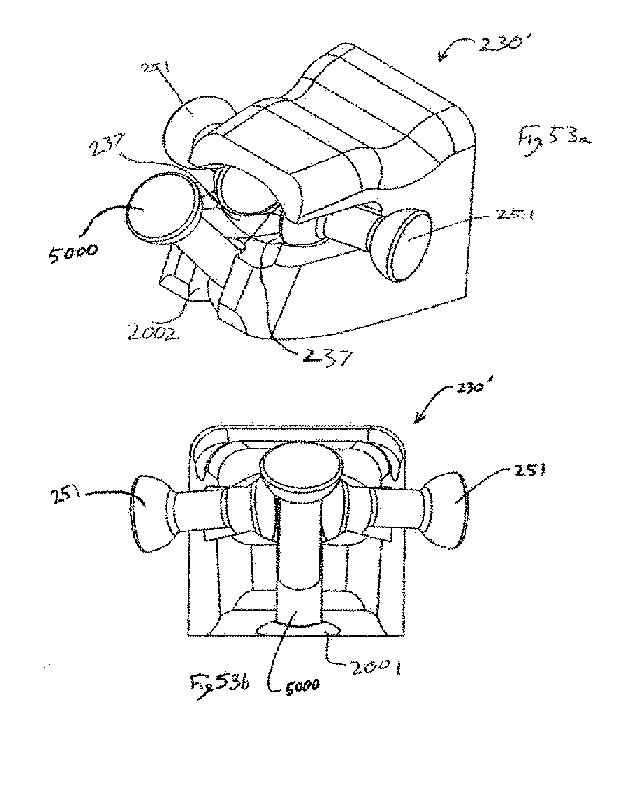

While FIGS. 49-52 depict the toe engagement portion 230' of the core 200' configured to accommodate a total of five links (e.g., four lateral links 250 and the vertical restraint link 5000), the toe engagement portion 230' may be configured to accommodate a total of three links. FIG. 53a illustrates an isometric view the toe engagement portion 230' according to an alternative implementation that accommodates a total of three links. FIG. 53b illustrates a front view of the alternative toe engagement portion 230'. In FIGS. 53a and 53b, the toe engagement portion 230' includes a single pair of recesses 237 that receive a pair of links 251, and a vertical restraint link ball holder 2001 and restraint link passage 2002 that receives the vertical restraint link 5000.

The recesses 237 may be configured similar to the upper and lower recesses 236, 238. In some embodiments, the recesses 237 may open at a 45 degree angle at a front plane and at a 90 degree angle relative to each other. However, in other embodiments the recesses 237 may open at a different angle relative to the front plane and be arranged at other angles relative to each other. The recesses 237 may be configured to receive a pair of links 251 (or links 250) that may be held within the recesses 237 due to the nature of the geometry of the recesses 237, or the links 251 may be held by a suitable mechanical assembly, such as a washer and fastener. The vertical restraint link 5000 is arranged in the toe engagement portion 230' in the manner described above in connection with FIGS. 50-51.

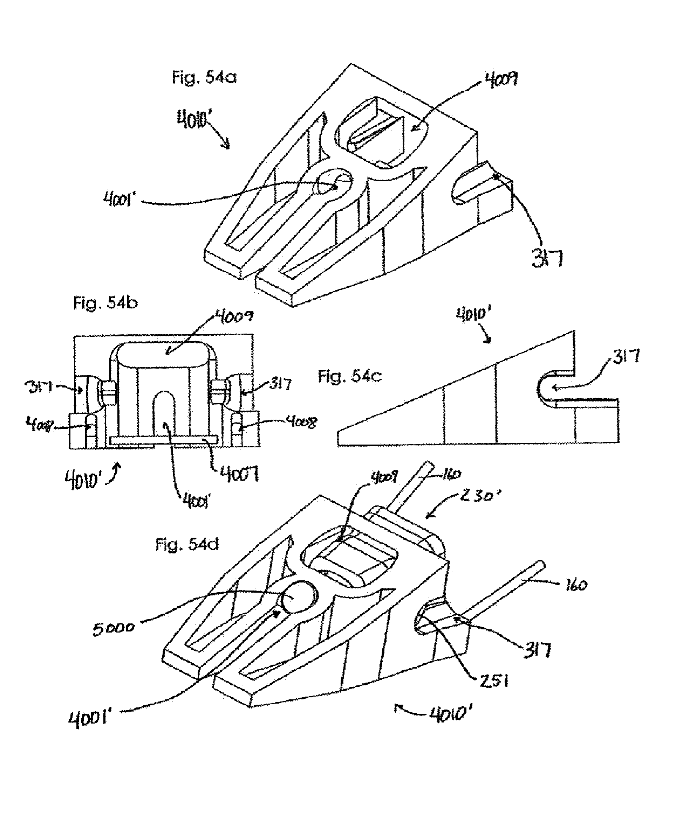

Turning to FIGS. 54a-54c, a toe assembly 4010' is illustrated in a front isometric view (FIG. 54a), a rear view (FIG. 54b) and a right side view (FIG. 54c). The toe assembly 4010' is configured to receive three links: the vertical link 5000 and two lateral links 250/251. The links may extend between the toe assembly 4010' and the toe engagement portion 230' illustrated in FIGS. 53a-53b.

The toe assembly 4010' includes recesses 317 arranged on a left and right rear portion. The recesses may have corresponding dimensions allowing movement of the lateral links 250/251 in the toe assembly 4010'. The recesses 317 may be configured similar to one of recesses 314 or 316 described above in connection with the artificial foot 10.

A central recess 4001' is also formed in a rear portion of the toe assembly 4010'. The central recess 4001' is formed through a central, front portion of the toe assembly 4010 and extends towards a rear portion of the toe and defines a ball holder for receiving one end of the vertical link 5000. The central recess 4001' is configured to allow the vertical link 5000 to extend from the toe assembly 4010' towards the toe engagement portion 230' along the longitudinal axis of the artificial foot 11 at an angle, e.g., a 45 degree angle, so that the other end of the vertical link 5000 couples to the toe engagement portion 230' at an angle, e.g., a 45 degree angle.

The toe assembly 4010' includes one or more cross-member 4007. The cross members 4007 serve to brace the toe body against the stress of, and resisting the deformation of, the inwardly directed forces of the lateral links interacting with the toe.

The toe assembly 4010' defines a bushing opening 4008 at a rear, bottom portion. The bushing opening 4008 extends from a bushing recess (not shown) defined in a bottom portion of the toe assembly 4010 to the external surface of the rear portion of the toe assembly (see FIG. 54b). The bushing opening 4008 enables the tension/limit members 160 to be seated in the bushing recess at the bottom portion of the toe assembly 4010, to pass through the bushing opening 4008, exit a back surface of the toe assembly 4010' and extend to the talus 100 for coupling with the toe spring carrier 270. The structure and function of the area of the toe assembly 4010' defining the busing opening 4008 may be similar to certain structures and functions of the bushing 1000 described below.

The toe assembly 4010' also defines a central opening 4009 at a top rear portion of the toe assembly proximate the central recess 4001'. The opening 4009 allows some movement of the toe engagement portion 230' through the opening 4009 during operation of the artificial foot 11. FIG. 54d illustrates the toe assembly 4010' operatively coupled with the toe engagement portion 230' via the lateral links 251 and the vertical link 5000. The tension/limit members 160 extend from the toe assembly 4010' to the talus 100 (not shown) via the busing openings 4008.

Returning to FIGS. 42-46, the figures illustrate the core 200' assembled to the toe 4000, with the vertical restraint link 5000 forming a vertical link and links 250 forming lateral links that movably couple the toe engagement portion 230' of the core 200' with the toe 4000. The vertical restraint link 5000 provides a portion of the joint 14' (e.g., the toe joint) to limit movement of the toe 4000 vertically relative to the core 200' during use of the artificial foot 11. For example, the vertical restraint link 5000 may restrain the toe 4000 from upward translation relative to the core 200'.

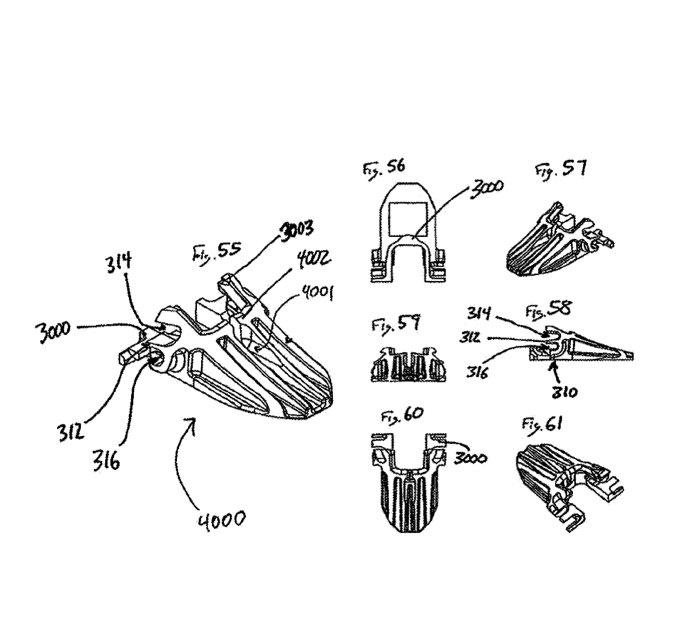

Turning to FIGS. 55-61, illustrated is the toe 4000 and the toe insert 3000 of the artificial foot 11 in isolation, various features are described without obstruction. FIG. 55 is a right isometric view of the toe 4000 and the toe insert 3000 of the artificial foot 11. FIG. 56 is a bottom view of the toe 4000 and the toe insert 3000 of the artificial foot 11. FIG. 57 is left isometric view of the toe 4000 and the toe insert 3000 of the artificial foot 11. FIG. 58 is a right side view of the toe 4000 and the toe insert 3000 of the artificial foot 11. FIG. 59 is a front view of the toe 4000 and the toe insert 3000 of the artificial foot 11. FIG. 60 is a top view of the toe 4000 and the toe insert 3000 of the artificial foot 11. FIG. 61 is a rear isometric view of the toe 4000 and the toe insert 3000 of the artificial foot 11.

The toe 4000 includes a central recess 4001 formed in a rear portion of the forwardly extending dorsal portion of the toe 4000. The walls of the central recess 4001 define a ball holder and an opening through the top surface 4002 of the toe 4000. The ball holder is configured to hold one end of the vertical restraint link 5000 (e.g., a spherical end) and the opening at the top surface 4002 is configured to allow the centralized cylindrical portion of the vertical restraint link 5000 to extend through the opening. In a neutral position of the vertical restraint link 5000, its centralized cylindrical portion is arranged at about a 45 degree angle with respect to the bottom plantar surface of the toe 4000 and extends along the longitudinal axis of the artificial foot 11. The toe insert 3000 of the toe 4000 is described below.

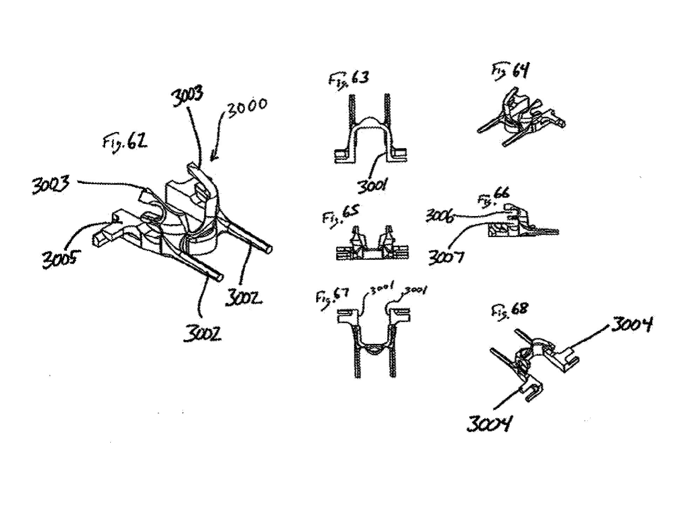

Turning to FIGS. 62-68, illustrated is the toe insert 3000 of the artificial foot 11 in isolation, various features are described without obstruction. FIG. 62 is a right isometric view of the toe insert 3000 of the artificial foot 11. FIG. 63 is a bottom view of the toe insert 3000 of the artificial foot 11. FIG. 64 is a left isometric view of the toe insert 3000 of the artificial foot 11. FIG. 65 is a front view of the toe insert 3000 of the artificial foot 11. FIG. 66 is a right side view of the toe insert 3000 of the artificial foot 11. FIG. 67 is a top view of the toe insert 3000 of the artificial foot 11. FIG. 68 is a rear isometric view of the toe insert 3000 of the artificial foot 11.

The toe insert 3000 provides strength to the toe 4000, and may be made of a metal such as steel, aluminum, nickel and super alloys. The toe insert 3000 includes an inner circumferential portion 3001, legs 3002, upper arms 3003, lateral flange 3004, lower arms 3005, upper link recess 3006 and lower link recess 3007. The toe insert 3000 is generally fabricated and plastic is overmolded over and around portions of the toe insert 3000 to form the toe 4000. For example, the toe insert 3000 may be placed in a mold and plastic is overmolded to form the toe 4000. The overmolded toe 4000 may be formed of a bearing grade material that provides the assembly with surface bearing properties such as derlin AF, Torlon, PEEK and PEEK blends (e.g., blends with teflon and graphite), and the like.

An inner circumferential portion 3001 of the toe insert 3000 is configured to surround the rounded lower front edge 234b of the toe engagement portion 230' of the core 200'. The inner portion 3001 may restrain lateral movement of the toe insert 3000 and therefore the toe 4000. When overmolded with the toe 4000, the inner portion 3001 may be exposed at least at a portion facing the core 200'. The configuration of the inner portion 3001 may be modified to allow for tight or relatively more forgiving tolerances, thereby allowing more or less movement of the toe insert 3000 relative to the core 200'.

The legs 3002 of the toe insert 3000 extend into the overmolded toe 4000 to provide additional rigidity to the assembly.

The upper arms 3003 of the toe insert 3000 extend towards the curved or rounded upper front edge 234a of the toe engagement portion 230' and are configured to guide movement of the toe 4000 and to restrain the toe 4000 from lateral movement as the arms 3003 abut the external surface 2004 of the toe engagement portion 230'. The terminal ends of the upper arms 3003 may be exposed from the overmolded portion of the toe 4000 at least at a portion facing the core 200', and the upper arms 3003 may be arranged substantially flush with the overmolded portion of the toe 4000 extending laterally from the upper arms 3003. The upper arms 3003 and the inner portion 3001 together may serve to guide and limit the toe 4000 as it moves relative to the core 200'. According to some embodiments, the upper arms 3003 may extend above the top surface of the toe 4000 to deflect stress exerted on the toe 4000 in the region associated with the upper arms 3003. See e.g., region of toe 4000 proximate upper arms 3003 in FIG. 55.

The lateral flanges 3004 of the toe insert 3000 are configured to receive or mate with the bushing 1000, described below.

The lower arms 3005 of the toe insert extend along the sidewalls of the core 200' beyond the toe engagement portion 230' (e.g., the lower arms 3005 extend beyond the overmolded portion of the toe 4000). Upon plantar flexion rotation about second joint 14', the lower arms 3005 may abut the external side walls 212 of the core 200'.

The upper link recess 3006 and the lower link recess 3007 are configured to receive the lateral links 250, and the overmolded portion of the toe 4000 may be formed around the external outer edges of the upper link recess 3006 and lower link recess 3007. Accordingly, the upper link recess 3006 and the lower link recess 3007 in combination with the upper pair of recesses 314 and the lower pair of recesses 316 may have corresponding dimensions allowing movement of the lateral links 250 in the toe 4000. In operation, as the second joint 14' moves into dorsiflexion, the toe assembly 4010 (i.e., the toe 4000, toe insert 3000 and bushing 1000) moves relative to the core 200', and the links 250 rotate and exert compressive forces on the upper link recess 3006 and the lower link recess 3007, e.g., the links 250 move laterally towards the longitudinal axis of the core 200' such that the end of the links coupled to the toe insert 3000 are pulled inwardly, and the rigid construction of the upper link recess 3006 and the lower link recess 3007 allows the toe assembly 4010 (e.g., toe 4000, toe insert 3000 and the bushing 1000) to withstand the compressive forces.

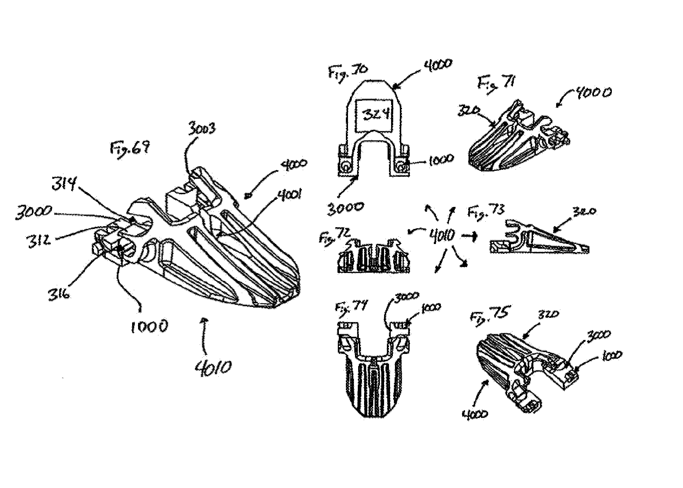

Turning to FIGS. 69-75, illustrated is the toe 4000 and the toe insert 3000 assembled with the bushing 1000 of the artificial foot 11. The assembly of the toe 4000, toe insert 3000 and the bushing 1000 may also be referred to as the toe assembly 4010. FIG. 69 illustrates a right isometric view of the toe assembly 4010. FIG. 70 is a bottom view of the toe assembly 4010. FIG. 71 is a left isometric view of the view of the toe assembly 4010. FIG. 72 is a front view of the toe assembly 4010. FIG. 73 is a right side view of the toe assembly 4010. FIG. 74 is a top view of the toe assembly 4010. FIG. 75 is a rear isometric view of the toe assembly 4010.

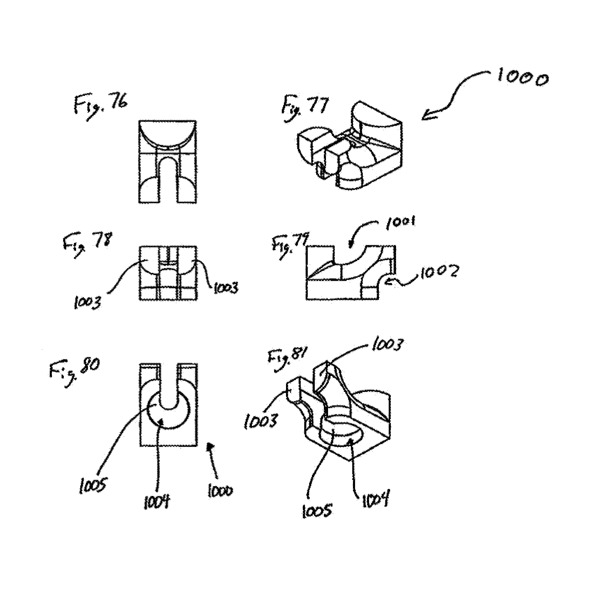

The bushing 1000 generally movably couples the toe assembly 4010 with the talus body 100 by way of the tension/limit members 260 when the artificial foot 11 is assembled. More specifically, the bushing 1000 serves to operably couple the toe 4000 and the spring carrier 270 arranged in the talus body 100 by way of the spherical bushings 264 of the tension/limit members 260. Two bushings 1000 are provided on each the left and the right side of the toe assembly 4010 (See, e.g., FIG. 74), and each bushing 1000 includes slots 1001 and 1002 that may be configured to couple with the lateral flange 3004 of the toe insert 3000. Additional details of the bushing 1000 are provided below in connection with FIGS. 76-81.

Turning to FIGS. 76-81, illustrated is the bushing 1000 for use in the toe assembly 4010 of the artificial foot 11 in isolation, various features are described without obstruction. FIG. 76 is a top view of the bushing 1000 of the artificial foot 11. FIG. 77 is an isometric view of the bushing 1000. FIG. 78 is a back view of the bushing 1000. FIG. 79 is a right side view of the bushing 1000. FIG. 80 is a bottom view of the bushing 1000. FIG. 81 is another isometric view of the bushing 1000.

The bushing 1000 may be a rigid unitary piece formed of a material that imparts strength to the toe 4000, and may be made of steel, aluminum, nickel, super alloys, and the like. The bushing 1000 may additionally include bearing components made of bearing grade materials, described further below.

As stated above, the bushing slots 1001 and 1002 are configured for insertion into the lateral flanges 3004 of the toe insert 3000. However, the bushing slots 1001 and 1002 may alternatively be configured to be received by pockets formed in the toe insert 3000. The back side of the bushing 1000 may be configured to be inserted or securely coupled to the toe insert 3000 by way of fastening means such as screws, bolts, rivets and the like.

At a back end of the bushing 1000 (e.g., FIG. 78), two arms 1003 extend towards the front of the bushing 1000 and converge into a spherically-shaped retaining member 1004 for receiving and retaining the spherical bushing 264 of the tension/limit member 260. The retaining member 1004 is illustrated in FIGS. 80 and 81, which illustrate the retaining member 1004 of the bushing 1000 defining a spherically-shaped recess that holds the spherical bushing 264 of the tension/limit member 260. At the bottom end of the bushing 1000 the spherical bushing 264 (FIG. 47a) is received at a bearing surface 1005 that contacts the spherical bushing 264 allowing for smooth operation of the artificial foot 11. The bearing surface 1005 may be unitarily formed with the bushing 1000 or the bearing surface 1005 may be provided as a bearing insert formed of a bearing grade material.

The assembly of the bushing 1000 with the toe insert 3000 and the toe 4000 may be configured so that the cable of the tension/limit member 260 exits the bushing 1000 in a straight line without bending. More specifically, the arms 1003 defining an opening in the bushing 1000 configured to allow the cable or cylindrical portion of the tension/limit member 260 to extend out of the toe assembly 4010 linearly in the direction of the talus body 100. An exit angle of the tension/limit member 260 exiting from the toe assembly 4010 may match an entrance angle of the tension/limit member 260 entering the spring carrier assembly 270. By providing longitudinal channels 228 in the core that match the exit angle of the bushing 1000, and therefore the tension/limit member 260 exiting therefrom, the end of the tension/limit member 260 coupled to the bushing 1000 may be substantially unstrained at the toe 4000. The other end of the tension/limit member 260 may be coupled to and operate in connection with the toe spring carrier assembly 270 in the manner described above in relation to the artificial foot 10. As a result, in operation of the artificial foot 11, the linearly extending tension/limit member 260 enables the toe 4000 to pivot relative to the core 200' while the tension/limit member 260 slides relative to the talus body 100 within the spring carrier assembly 270 without bending the tension/limit member 260.

It will be understood that, while the toe 4000, toe insert 3000 and the bushing 1000 of the toe assembly 4010 are described separately, the toe assembly 4010 may be configured as a single piece, unitarily constructed. For example, the toe assembly 4010 may include structures corresponding the toe 4000, toe insert 3000 and bushing 1000, but the toe assembly 4010 may be entirely formed through an injection molding process. FIGS. 54a-54c illustrate a toe assembly 4010' constructed of a single piece of material, such as an injection molded plastic or fabricated material having bearing properties. The toe assembly 4010' includes structures corresponding to the busing 100, toe insert 3000 and toe 4000 that enable the features of the toe insert 4010' to operate in the manner described below in connection with the artificial foot 11. In some implementations the toe assembly 4010 may be formed of two pieces. For example, the toe insert 3000 and the bushing 1000 may be unitarily formed and then overmolded with the toe 4000. In another example, the bushing 1000 is inserted into an injection mold and the toe assembly 4010 is formed by overmolding structures corresponding to the toe insert 3000 and the toe 4000. It will be appreciated that the toe assembly 4010 formed as a unitary piece may be formed of a bearing grade material or a metal, for example. When formed of two components, the toe assembly 4010 may be fabricated from metal as well as a bearing grade material, two metals, or two bearing grade materials, for example.

As will be appreciated, the talus body 100 may be operatively coupled with the core 200' via a first joint 12', and the core 200' may be operatively coupled with the toe 4000 via a second joint 14'. Various modifications to the second joint 14' are described above in connection with the toe assembly 4010.

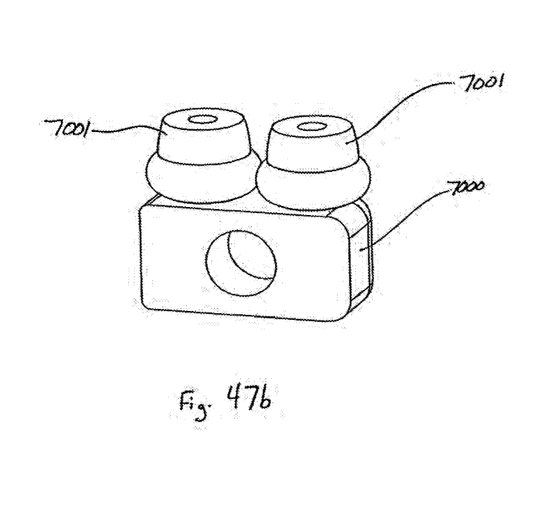

Turning to the first joint 12', the artificial foot 11 includes pivot bearings 7000 provided on the lateral axle 124a of the bearing 124 (FIG. 47a). The bearing recesses 224' of the core 200' are configured to accommodate the pivot bearings 7000. Returning to FIG. 41, with respect to the first joint 12' of the artificial foot 11, certain structural and functional similarities are provided between this joint and the first joint 12 of the artificial foot 10. In particular, the first joint 12' includes pivot bearings 7000, which are similar to the bearings 226 described above in connection with FIG. 7. For example, as shown in FIGS. 47a and 47b, the pivot bearings 7000 include an interior surface configured to couple to the lateral axle 124a of the talus bearing 124. The internal surface of the pivot bearing 7000 slides with the lateral axle 124a in the pitch direction (e.g., in the context of roll, pitch and yaw directions). An external surface of the pivot bearings 7000 is configured to contact the core pockets formed by bearing recess 224' of the mid-foot bearing portion 220' of the core 200'. Flat surfaces on the outside of the pivot bearings 7000 slide along the surfaces of the core 200' in the yaw and roll directions. The pivot bearings 7000 may be fabricated of bearing grade material, described above. When coupled, the lateral axle 124a, pivot bearings 7000 and bearing recesses 224' thus may allow constrained relative rotation and translation between the talus body 100 and the core 200'. The bearing recesses 224' may allow for a desired degree of movement of the respective pivot bearings 7000 within the bearing recesses 224'.

In addition, the pivot bearings 7000 may be fabricated with shock absorbing materials such as urethane (e.g., a viscoelastic urethane polymer), a rubber coating, or another bumper material. The pivot bearings 7000 slide along the surface of the bearing recess 224' during the heel strike and plantar flexion motions. Generally, motion of the talus body 100 relative to the core 200' causes the talus body 100 nose to move upwards resulting in the pivot bearings 7000 contacting the top of the bearing recess 224'. The pivot bearings 7000 may form or include the shock absorbing material, which may impart sound deadening properties to the first joint 12' as the pivot bearings 7000 contact the bearing recess 224'. In this way, the pivot bearings 7000 are configured to absorb a force generated by a movement of the talus body 100 against to the core 200' and prevents the talus body 100 from making clicking noises as the artificial foot 11 is used in the walking motion. In the case of providing pivot bearings 7000 having a shock absorbing features, the bearing recess 224' may be configured to receive such modified pivot bearings.

In some implementations, the pivot bearings 7000 may be coupled to one or more bumpers 7001 (FIGS. 47a and 47b) mounted on an external surface of the pivot bearings 7000 and the bumpers 7001 may absorb a shock generated by a movement of the talus body 100 against to the core 200'. For example, during operation of the artificial foot 11, the bumpers 7001 may serve to cushion and/or limit upward movement of the talus body 100. The bumpers 7001 may be mounted on a top portion of the pivot bearings 7000, e.g., by mechanical fastening, sonic welding or integrally forming with the pivot bearings 7000, may have a prism-like configuration or tube-like configuration, and be made of a shock absorbing material like urethane, for example. The bumpers 7001 may have a similar configuration to the bumpers 130, while in some implementations, the bumpers 7001 may be relatively smaller, (e.g., shorter or narrower) than bumpers 130. The roll and upward motion of the talus 100 relative to the core 200' may have a torque response curve based on the hardness of the pivot bumpers 7001 as well as the bumpers 130.

In alternative implementations, the bumper(s) 7001 may be mounted on the core 200, as appropriate or desired. For example, the bearing recesses 224' may be modified to include bumper 7001 or shock absorbing material, and/or may include additional bumpers.

In some implementations, the core 200' may be modified to accommodate the bumpers 7001 and/or the pivot bearings 7000, and the modifications to the core 200' may provide structural reinforcement to the core 200'. Structural reinforcement may include reinforcement members such as bolts, screws, rivets, and the like, extending across the core 200' in the area of the bearing recesses 224', and the talus body 100 may additionally form a through bore for allowing the reinforcement member to pass through the talus body 100, thereby allowing the reinforcement member couple the sides of the core 200' while extending through the talus body 100.

According to certain implementations, the core 200' may additionally be provided with a core bearing insert in an interior of the core 200'. The core bearing insert may provide an interface (e.g., a tribological interface) between the nose of the talus body 100 and the core 200'. The core bearing insert may be fabricated of bearing grade material, described above. In this implementation, the core 200' may be made of urethane or other non-bearing grade materials, while the a portion of the core components, e.g., the core bearing insert provides a bearing surface constructed of bearing grade material for contacting the shaped side bearing surfaces 122a of the front end 122 of the talus body 100.

As explained further below, generally, in operation of the artificial foot 11, the torque response curve for the second joint 14' is isolated from the range of motion for the toe 4000 relative to the core 200' and the torque response curve for the first joint 12' is isolated from the range of motion of the core 200' relative to the talus 200'. However the torque response curve of the second joint 14' is responsive to the torque response curve of the first joint 12' due to the common use of the springs 278 of the toe spring carrier assembly 270.

Operation of the artificial foot 11 during walking movement may be described as follows. It will be understood that structures in the operation of the artificial foot 10 and references to FIGS. related to the artificial foot 10 may be applicable to the operation of the artificial foot 11. Beginning with heel strike, the heel strike portion 210 of the core 200' may absorb impact. The talus body 100 may move downward toward the heel strike portion 210 with the bumpers 130 (when provided) contacting the upper surface of the heel strike portion 210 to absorb the heel strike. The talus body 100 may rotate (counter-clockwise in FIG. 42) relative to the core 200' during downward movement of the talus body 100, moving the pivot bearings 7000 (FIG. 47a) to a top of the bearing recesses 224' of the core 200'.

The talus body 100 may then rotate about a virtual lateral axis in an opposite direction (clockwise in FIG. 42) relative to the core 200' until the artificial foot reaches a neutral (standing support) position. In the neutral position, the pivot bearings 7000 may return to a bottom of the bearing recesses 224'. Further in this position, the artificial foot may be supported via the bottom of the core 200' as a contact/support/load-bearing surface via the bumpers 130 of the talus 100.

From the neutral position, tibial progression in a forward direction causes the talus body 100 to rotate about the axle 124a (FIG. 47a) of the first joint 12', via the pivot bearings 7000. The coil springs 298 in the spring carrier assembly 290 (FIG. 7) are loaded against the upper bearing surface 116a and the coil springs 278 (FIG. 7) in the spring carrier assembly 270 (FIG. 47a) are loaded against the lower bearing surface 118a as the tension/limit member 260 is loaded.

Continued tibial progression forward causes heel rise or lift-off as well as rotation of the toe assembly 4010 relative to the core 200' at the second joint 14'. As the heel rises, the artificial foot 11 is supported by the toe engagement portion 230' of the core 200', with the toe assembly 4010 continuing to rotate up and around the toe engagement portion 230' in the dorsiflexion direction, as indicated by the arrow "D" in FIG 41, and due to the geometry of the toe assembly 4010, the links 250, 5000 are brought into tension between the toe assembly 4010 and the core 200'. The toe assembly 4010 moves as a rigid unit rotating on the links 250, and is restrained in the vertical direction by the vertical restraint link 5000. The toe assembly 4010 moves about an axis generally defined by the links 250 moving in the dorsiflexion direction D. This motion is opposed by the force of the springs 278 of the toe spring carrier 270 compressed between the talus body and the toe spring carrier 270. When the toe spring carrier 270 contacts the substantially planar lower bearing surface 118a of the lower portion 118 of the talus body 100, the tension/limit members 260 substantially limits the motion of the toe assembly 4010 in the dorsiflexion direction D.

Motion of the toe assembly 4010 around the axes of the artificial foot 11 may be limited by the interference between the toe insert 3000 and the core 200' via surfaces of the toe engagement portion 230' and the side walls 212. For example, roll and yaw movement allowed between the toe assembly 4010 and the core 200' may be controlled by the distance between the exterior surface 2004 of the toe engagement portion 230' and the inner surface 3001 of the toe insert 3000. As will be appreciated, a certain amount of motion between the toe insert 3000 and the exterior surface 2004 is desirable to allow the links 250, 5000 and the tension/limit members 160 to engage the toe with the core 200' and the talus body 100. Continuing with this example, the pitch movement of the toe assembly 4010 is based on a degree of movement allowed by the links 250, 5000 coupling the toe assembly 4010 to the core 200'.

According to certain implementations, the range of motion of the second joint 14' is based on the degree of motion the toe assembly 4010 is able to rotate about the two or four lateral links 250/251 as well as the vertical link 5000. The planes about which the toe assembly 4010 moves relative to the core 200' is separate from the tension/limit members 260, and thus the talus body 100, the spring carrier assembly 270 and the core spring carrier assembly 290 do not affect the degree of the range of motion engaged in by the toe assembly 4010. As a result, the range of motion of the toe assembly 4010 is separate from the torque response engaged in by the joints of the artificial foot 11. For example, this allows the range of motion of the second joint 14' to be easily tunable for individual needs and to adjust the spring rate of the torque response curves of the second joint 14'.

According to certain implementations, the range of motion of the toe assembly 4010 at the second joint 14' is based on the degree of motion the toe assembly is able to rotate and the gap between toe spring carrier 270 and the surface 118a. As the second joint 14' moves into dorsiflexion, the tension member 260 causes compression of the springs 278, decreasing the gap between toe spring carrier 270 and the talus body surface 118a. When this gap narrows to the point that the toe spring carrier 270 is in contact with talus body surface 118a, the pitch direction of the second joint 14' is effectively at the maximum of this range of motion, while both the roll and yaw directions continue to be compliant and conform to surfaces beneath toe assembly 4010.

According to certain implementations, as the artificial foot 11 operates, the first joint 12' engages in a torque response, e.g., torque as a function of angular relative motion as the mid-foot joint engages in pivoting movements that create torque. As described above, during use, when the talus body 100 is rotated forwardly relative to the core 200', the talus body 100 rotates about the axle 124a (FIG. 47a) of the first joint 12', via the pivot bearings 7000. The coil springs 298 in the spring carrier assembly 290 (FIG. 7) are loaded against the upper bearing surface 116a and the coil springs 278 (FIG. 7) in the spring carrier assembly 270 (FIG. 47a) are loaded against the lower bearing surface 118a as the tension/limit member 260 is loaded. This pivoting and loading movement as the artificial foot operates 11 in the manner described above provides a torque response curve for the artificial foot 11.

After the first joint 12/12' has reached the maximum of the pitch range of motion by closing the gap between spring carrier 290 and upper bearing surface 116a, the foot rolls forward onto the curved surface 234b of core 200', forcing the second joint 14/14' into dorsiflexion, further closing the gap between the toe spring carrier 270 and the lower bearing surface 118a of the talus body 100. This dorsiflexion motion continues until either toe spring carrier 270 is in contact with lower bearing surface 118a or until the lateral links 250 and the vertical link 5000 interact with the toe attachment portion 230 and the toe assembly 4010. Either of these two modes of operation may serve to limit the range of motion of the second joint 14/14' in the pitch direction depending on the configuration of the relative lengths of the tension members 280 and 260, the relative gaps between spring carrier assemblies 290 and 270 and their respective bearing surfaces 116a and 118a, and the angle and irregularity of the terrain underneath either artificial foot 10 or artificial foot 11.

As the contralateral leg has heel strike, a substantial portion of the weight is removed from the artificial foot 10 or 11, allowing the springs 298 and 278 to recoil, projecting the artificial foot 10 or 11 into a forward and somewhat upward trajectory of "swing phase" by virtue of having a reaction force vector that points upward and between the first and second joints, 12/12' and 14/14'. Also, as tension members 260 span both the first and second joints 12/12' and 14/14', there is an energetic instability that causes a faster response than if tension member 260 only crossed the second joint 14/14'.

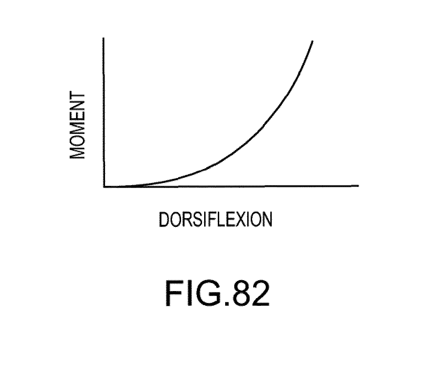

With respect to the artificial foot 10 and the artificial foot 11, because the first joint 12/12' operates to mimic the "human like" torque response curve (FIG. 82), the first joint 12/12' functions analogously to the human subtalar joint and the ankle joint combined. The torque response curve generated by the motion of the first joint 12/12' in the direction of travel generates is depicted in FIG. 82. The first joint 12/12' initially moves easily for the first few degrees around all three axes (roll, pitch and yaw movements), e.g., as the artificial foot conforms to irregularities in terrain. The motion in the roll and yaw directions are then blocked by the contact of talus surfaces 112a and core surfaces 222a. The motion in the pitch direction becomes progressively stiffer until the maximum range of motion is reached, in an approximation of the natural human anatomy. The torque response curve is dictated by the placement of the fixed end of tension member 280 at recess 216, and the placement of the bearing surface 116a, both relative to the location of the axle 124a. Also, as described below, the spring rate of springs 298 and 278 also dictate the steepness of the torque response curve of this joint. The motion of the first joint 12/12' and the artificial foot 10/11 generally then simulates the vaulting of the arch in walking and becomes progressively stiffer until reaching a limit of the range of motion. The motion of the first joint 12/12' is cushioned using the pivot bearings 226/7000 and any additional bumpers 7001 or core inserts. The spring rate of the torque response curve is tunable by providing elastomeric members or coil springs (e.g., coil springs 278 and/or coil springs 298) having one or more selected resistances. In addition, as discussed above, the cavities 296 of the core spring carrier assemblies 270, 290 may be configured to hold the spring elements at different depths such that not all spring elements are compressed at the same time during operation of the first joint 12/12'. For example, providing one or more larger coil springs 298 that contact the upper bearing surface 116a of the talus body 100, a larger overall stiffness of the joint 12/12' is provided. If smaller coil springs 298 contact the bearing surface 116a first, there is a smaller initial stiffness of the joint 12/12'. Thus, the length and stiffness of the coil springs 278, 298 may also be used to "tune" the torque response curve. Similarly, by tightening the attachment hardware on tension bolt 280, a smaller initial distance may be set between the bottom edge of spring carrier 290 and bearing surface 116a, providing a larger initial stiffness, along with a smaller maximum range of motion in the pitch direction. A spring carrier 92 with deeper pockets 296 will also provide a smaller gap between carrier 290 and bearing surface 116a, providing a smaller range of motion in the pitch direction of the first joint 12/12', with out increasing the initial stiffness of the joint. Similar adjustments can be made for the spring carrier 270/bearing surface 118a gap.

In operation, embodiments may provide improved movements for an artificial foot device. For example, if the foot encounters an uneven surface, such as a relatively small rock, and a portion of the artificial foot is placed at an angle about its longitudinal axis by such, the limited movement provided in one or both of the joints about the longitudinal axis may allow compliance to avoid translating at least part of the angle to the attachment pylon and/or the user's leg and/or body. In particular, if the uneven surface occurs under the toe and/or the core-assembly, the mid-foot joint may permit constrained movement to accommodate the resulting angle. If the uneven surface occurs under the toe, the second joint 14' (e.g., toe joint) may permit constrained movement to accommodate the resulting angle. In either case the other of the two joints may also contribute to accommodation of the resulting angle.

Also, the limited movement provided in one or both of the joints about a substantially vertical axis (e.g., substantially perpendicular to the longitudinal axis) may allow compliance to allow a user to change direction of travel while ambulating. Although both joints may contribute to the compliance, in some embodiments, the second joint 14' (e.g., toe joint) may provide a primary or sole contribution to the compliance. In some embodiments, the compliance may be provided without substantial energy storage, for example, to avoid undesirable backlash from the change in direction. Similarly, in some embodiments, the mid-foot joint may provide a primary or sole contribution to the compliance.

It should be understood that the various tension members may be of varying degree of flexibility and/or elasticity. Various materials may be employed for the tension members, as well as for the discontinuous members of the artificial foot. The materials may be sufficiently rigid, strong, and/or flexible, as appropriate for the function of the particular member. In some aspects, flexibility of a particular tension member the may not be important, as long as the design maintains the member loaded primarily in tension. The weight of the user and the selected use by that user may be considered when selecting materials. For example, stronger materials may be required when the user intends to jump and land hard as compared to when the user merely intends to walk. Useful materials for discontinuous members may include metals and/or plastics. Useful materials for tension members may include steel wire rope and aramid fiber ropes. Metal, ceramic and/or plastic bearings may be also useful in the practice of the invention. Preferably, the members of the joints and foot parts of the invention may be made from aluminum (e.g., 7075 T6), steel wire rope, tool steel, plastics and/or other suitable materials based on desired rigidity, flexibility, strength, toughness and the like.

While certain exemplary embodiments have been described above in detail and shown in the accompanying drawings, it is to be understood that such embodiments are merely illustrative of and not restrictive of the broad invention. In particular, it should be recognized that the teachings provided herein apply to a wide variety of systems and processes. It will thus be recognized that various modifications may be made to the illustrated and other embodiments described herein, without departing from the broad inventive scope thereof. In view of the above it will be understood that the invention is not limited to the particular embodiments or arrangements disclosed, but is rather intended to cover any changes, adaptations or modifications which are within the scope and spirit of the disclosure provided herein.

Furthermore, one or more aspects of the following numbered clauses may describe and relate to further aspects or features within the context of the present teaching:
1. An artificial foot device, comprising:
   a toe pivotably coupled with a core at a joint;
   a vertical restraint link extending across the joint that couples the toe with the core;
   wherein the vertical restraint link limits motion of the toe in a vertical direction relative to the core.
2. The foot device of clause 1, wherein
   a first end of the vertical restraint link couples to the toe at a central recess formed through a top portion of the toe; and
   a second end of the vertical restraint link, opposite the first end, couples to the core at a recess formed at a bottom portion of the core.
3. The foot device of clause 1, wherein the vertical restraint link extends along a longitudinal axis of the foot.
4. The foot device of clause 3, wherein in a neutral position of the foot device, the vertical restraint link extends across the joint at about a 45 degree angle.
5. The foot device of clause 1, further comprising a first lateral link member and a second lateral link member coupling the toe with the core, the first lateral link member on a first lateral side of the device and the second lateral link member on a second lateral side of the device.
6. The foot device of clause 5, wherein in a neutral position of the foot device, the toe rotates about the lateral links and is restrained in a vertical direction by the vertical restraint link.
7. The foot device of clause 1, wherein
   the toe surrounds a portion of the core; and
   the toe limits rotational motion of the joint by interference between the toe and the core.
8. An artificial foot device, comprising:
   a talus body;
   a core pivotably coupled with the talus body at a first joint;
   a toe pivotably coupled with the core at a second joint; and
   a tension member coupling the toe to the talus body.
9. The foot device of clause 8, wherein as the toe pivots about the second joint, the tension member linearly slides along a biasing assembly acting between the toe and the talus.
10. The foot device of clause 9, wherein
   the tension member comprises a rope-like member; and
   the biasing assembly comprises at least one spring or elastomeric member; and
   the rope-like member extends linearly between the toe and the biasing assembly coupled to the talus body.
11. The foot device of clause 10, wherein the biasing assembly is arranged in an interior of the talus body.
12. The foot device of clause 8, wherein the tension member limits rotational movement of the toe relative to the core.
13. An artificial foot device, comprising:
   a talus body;
   a core pivotably coupled with the talus body at a first joint; and
   a biasing assembly acting between the talus and the core at the first joint;
   wherein the talus body pivots about the first joint and causes the biasing assembly to provide a torque response for the artificial foot device.
14. The foot device of clause 13, wherein
   the first joint comprises a lateral axle extending from the talus body;
   pivot bearings are coupled to the lateral axle;
   the talus body pivots about the pivot bearings; and
   a force generated by a movement of the talus body relative to the core is cushioned by the pivot bearings.
15. The foot device of clause 14, wherein the core comprises bearing recesses configured to accommodate the pivot bearings.
16. The foot device of clause 15, wherein the bearing recesses are configured such that the talus body pivots relative to the core about the pivot bearings in a constrained relative rotation and translation.
17. The foot device of clause 14, further comprising one or more bumpers coupled to the pivot bearings for absorbing the force generated by the movement of the talus body relative to the core.
18. The foot device of clause 13, wherein the biasing assembly comprises a carrier assembly and one or more springs or elastomeric members.
19. The foot device of clause 18, wherein the biasing assembly is coupled to the talus body by a tension member.
20. The foot device of clause 18, wherein the biasing assembly is biased against a bearing surface of the talus body to provide the torque response.

## Claims

1. An artificial foot device, comprising:
a talus body (100);
a core (200/200') pivotably coupled with the talus body (100) at a first joint (12/12'); and
a biasing assembly (290) acting between the talus and the core at the first joint; wherein
the talus body pivots about the first joint and causes the biasing assembly to provide a torque response for the artificial foot device;
the first joint comprises a lateral axle (124a) extending from the talus body;
pivot bearings (7000) are coupled to the lateral axle; and
the talus body pivots about the pivot bearings and the pivot bearings slide along the core.

2. The foot device of claim 1, wherein
the biasing assembly limits the range of motion of the first joint; and
the biasing assembly is coupled to the core by a tension member and is operated by the motion of the talus body.

3. The foot device of claim 2, wherein the core comprises bearing recesses configured to accommodate the pivot bearings.

4. The foot device of claim 3, wherein the bearing recesses are configured such that the talus body pivots relative to the core about the pivot bearings in a constrained relative rotation and translation.

5. The foot device of claim 2, further comprising one or more bumpers coupled to the pivot bearings for absorbing the force generated by the movement of the talus body relative to the core.

6. The foot device of claim 1, wherein the biasing assembly comprises a carrier assembly and one or more springs or elastomeric members.

7. The foot device of claim 5, wherein the biasing assembly is biased against a bearing surface of the talus body to provide the torque response.

8. The artificial foot device of claim 1, further comprising:
a toe pivotably coupled with the core at a second joint; and
a vertical restraint link extending across the second joint that couples the toe with the core; wherein
a first end of the vertical restraint link couples to the toe at a central recess formed through a top portion of the toe;
a second end of the vertical restraint link, opposite the first end, couples to the core at a recess formed at a bottom portion of the core; and
the vertical restraint link limits motion of the toe in a vertical direction relative to the core.

9. The foot device of claim 8, wherein
the vertical restraint link extends along a longitudinal axis of the foot; and
in a neutral position of the foot device, the vertical restraint link extends across the joint at about a 45 degree angle.

10. The foot device of claim 8, further comprising a first lateral link member and a second lateral link member coupling the toe with the core, the first lateral link member on a first lateral side of the device and the second lateral link member on a second lateral side of the device.

11. The foot device of claim 10, wherein in a neutral position of the foot device, the toe rotates about the lateral links and is restrained in a vertical direction by the vertical restraint link.

12. The foot device of claim 8, wherein
the toe surrounds a portion of the core; and
the toe limits rotational motion of the joint by interference between the toe and the core.

13. The artificial foot device of claim 1, further comprising:
a toe pivotably coupled with the core at a second joint; and
a tension member coupling the toe to the talus body.

14. The foot device of claim 13, wherein
the tension member comprises a rope-like member; and
the biasing assembly comprises at least one spring or elastomeric member; and
the rope-like member extends linearly between the toe and the biasing assembly coupled to the talus body.

15. The foot device of claim 13, wherein the tension member limits rotational movement of the toe relative to the core.
